# EUROPEAN PATENT APPLICATION

(11) **EP 2 172 454 A1**
(43) Date of publication of application: **07.04.2010**
(21) Application number: 08791469.3
(22) Date of filing: 23.07.2008
(51) Int. Cl.: C07D 209/08, A61K 31/404, A61K 31/4184, A61K 31/4439, A61P 5/24, A61P 5/28, A61P 5/38, A61P 13/08, A61P 15/00, A61P 17/00, A61P 17/08, A61P 17/10, A61P 17/14, A61P 35/00, A61P 43/00, C07D 209/12, C07D 235/06, C07D 235/10, C07D 235/12, C07D 235/26, C07D 401/04

(54) **BENZIMIDAZOLE DERIVATIVE**

(30) Priority: 24.07.2007 JP 2007192242
(71) Applicant: Astellas Pharma Inc., Tokyo 103-8411 (JP)
(72) Inventor: KAKEFUDA, Akio, Tokyo 103-8411 (JP); KONDOH, Yutaka, Tokyo 103-8411 (JP); HIRANO, Masaaki, Tokyo 103-8411 (JP); KAMIKAWA, Akio, Tokyo 103-8411 (JP); ENJO, Kentaro, Tokyo 103-8411 (JP); FURUTANI, Takashi, Tokyo 103-8411 (JP)
(74) Representative: Bates, Philip Ian
(86) International application number: PCT/JP2008/063212
(87) International publication number: WO 2009/014150

(57) **Abstract**

Provided is a novel and excellent method for treating and/or preventing benign prostatic hyperplasia, prostate cancer, and the like based on selective inhibitory activity against 17βHSD type 5. It was found that an indole or benzimidazole derivative, where a nitrogen atom of the indole ring or benzimidazole ring is substituted with a phenyl group substituted with COOH, has a potent selective inhibitory activity against 17βHSD type 5 and may become an agent for treating and/or an agent for preventing a disease associated with 17βHSD type 5, such as benign prostatic hyperplasia, prostate cancer and the like, without accompanying adverse effects due to a decrease in testosterone; and the present invention has thus been competed.

## Description

### Technical Field

The present invention relates to an indole compound and/or a benzimidazole compound having a pharmacological activity and/or a pharmaceutically acceptable salt thereof. Further, the present invention relates to a pharmaceutical or a pharmaceutical composition containing the indole compound and/or benzimidazole compound and/or a pharmaceutically acceptable salt thereof described above as an active ingredient.

### Background Art

Benign Prostatic Hyperplasia (BPH) is a disease mainly occurring in elder males aged 50 years or above and accompanying urinary disorders, and its incidence rate increases with the age. The number of patients with BPH in Japan has been constantly increasing in recent years with the rapid aging of the population. BPH remarkably deteriorates the quality of life of the aged males due to urinary disorders, and it is an important disease in terms of medical economics since it is the most frequently diagnosed and treated disease in the medical field of urology.

It has been found that two factors, that is, direct urethral compression due to hypertrophy of the prostate (mechanical obstruction) and elevation of intraurethral pressure due to overcontraction of the prostatic smooth muscle via the sympathetic nerve (functional obstruction), are simultaneously involved in urinary disorders accompanying BPH. Drug therapy can deal with both of these mechanisms, and 5α-reductase inhibitors are mainly used for the mechanical obstruction and α1-sympatholytic agents (α1 blockers) are mainly used for the functional obstruction. 5α reductase inhibitors regress the prostate due to their anti-androgenic effect based on the suppression of the conversion of testosterone to 5α-dehydrotestosterone (DHT) which is a more potent androgen produced by a 5α-reductase. Only the prostatic epithelium regresses, however, and it takes a long period of time (several weeks to several months) for the drug efficacy to become apparent. On the other hand, since α1-blockers exert their drug efficacy swiftly after administration and are excellent in safety, α1-blockers are now the first-line agent for treating BPH. However, as a result of the long-term clinical studies, since a 5α-reductase inhibitor significantly delayed the transfer to invasive therapy as compared with the single use of an α1-blocker, and the like ("The New England Journal of Medicine", 2003, Vol. 349, p. 2387-2398), the usefulness of 5α-reductase inhibitors has recently been recognized again.

It has been considered that DHT in the prostate is produced by 5α-reductase from testosterone, which is produced in the testes and secreted endocrinologically to the prostate. It has been reported recently, however, that about half of DHT and its precursor, testosterone, in prostate, are synthesized from dehydroepiandrosterone (DHEA), a steroid derived from an adrenal, in cells of the prostate ("Frontier in Neuroendocrinology", 2001, Vol. 22, p. 185-212). This kind of sex hormone production system in the cells of the sex hormone target organs is called intracrinology.

It is difficult for 5α-reductase inhibitors to inhibit the local testosterone synthesis (intracrine testosterone synthesis) in the prostate. For example, it has been reported that the concentration of DHT in the prostate of the patients with BPH was decreased after the administration of finasteride, a 5α-reductase inhibitor, to about 20% of the concentration before the administration, while the concentration of testosterone, a precursor, in the prostate was inversely increased 4-fold ("The Journal of Urology", 1999, Vol. 161, p. 332-337). It means that although the 5α-reductase inhibitor has an effect of reducing DHT concentration in the prostate, it has no effect of reducing the concentration of testosterone in the prostate and instead elevates the concentration. Since testosterone has an androgen receptor binding activity of about the half of that of DHT, this local elevation of the concentrations of testosterone in the prostate is considered to be partly responsible for insufficient drug efficacy of finasteride for BPH.

Anti-androgen therapies using surgical castration and gonadotropin releasing hormone agonists are also used prostate cancer. These anti-androgen therapies have been reported to exert an insufficient effect of reducing the concentrations of testosterone in the prostate. For example, in patients with prostate cancer who receive the anti-androgen therapy, the concentration of testosterone in the blood decreased to about 10% of the concentration before the therapy, while the concentration of DHT in the prostate remained at about 50% ("The Journal of Clinical Endocrinology and Metabolism", 1995, Vol. 80, p. 1066-1071 It suggests that the concentration of testosterone in the prostate is also not sufficiently reduced. Further, androgen receptors were localized in nuclei also in a prostate cancer recurring after anti-androgen therapy (Hormone Refractory Prostate Cancer), and no significant difference was observed between the concentration of testosterone in recurrent prostate cancer tissues and that in the normal prostate ("Clinical Cancer Research", 2004, Vol. 10, p. 440-448). These reports strongly suggest that the effect of reducing the concentrations of testosterone in the prostate in existing therapeutic methods is quite insufficient for treating recurrent prostate cancer and that suppression of the testosterone synthesizing mechanism in the prostate, that is, intracrine testosterone synthesis in the prostate may be a new target of prostate cancer therapy.

Based on the known arts described above, since inhibitors of intracrine testosterone synthesis in the prostate have an effect of reducing the concentrations of testosterone in the prostate and no effect of reducing the concentrations of testosterone in the blood, the inhibitors are expected to be very attractive agent for treating BPH and/or an agent for treating prostate cancer, (1) which can reduce not only the concentration of testosterone but also the concentration of DHT in the prostate and (2) which can avoid the adverse effects due to the suppression of the concentration of the testosterone derived from testes in the blood.

17β-hydroxysteroid dehydrogenase (17βHSD) is essential for the biosynthesis of testosterone. There are several subtypes of 17βHSD. 17βHSD type 5 is highly expressed in a human prostate and increases of the expression were reported for prostate cancer and recurrent prostate cancer ("Steroids", 2004, Vol. 69, p. 795-801; and "Cancer Research", 2006, Vol. 66, p. 2815-2825). On the other hand, almost all the testosterone in the blood is produced by 17βHSD type 3 in testes and the expression of 17βHSD type 3 is rarely observed in other tissues including the prostate ("Nature Genetics", 1994, Vol. 7, p. 34-39). 17βHSD type 5 is thus considered to be responsible for the intracrine testosterone synthesis in the prostate and selective inhibitors for 17βHSD type 5 are expected to suppress intracrine testosterone synthesis in the prostate selectively. Further, since the contribution of 17βHSD type 5 has been pointed out also in estrogen-dependent tissues such as the mammary gland and the like, the selective inhibitors are expected to be effective for estrogen-dependent diseases such as breast cancer and the like ("Endocrine Reviews", 2003, Vol. 24, p. 152-182). In addition, since AKR1C3 (another name for 17βHSD type 5), which is a subtype of aldo-keto reductase (AKR), metabolizes Polycyclic Aromatic Hydrocarbon (PAH) to generate reactive oxygen species (ROS) ("The Journal of Biological Chemistry", 2002, Vol. 277, No. 27, p. 24799-24808) and since single nucleotide polymorphism (SNP) of AKR1C3 gene relating to oxidative stress correlates with a risk of lung cancer ("Carcinogenesis", 2004, Vol. 25, No. 11, p. 2177-2181), it is suggested that the activity of AKR1C3 in the lungs increases the risk of lung cancer via generation of ROS from PAH. Selective inhibitors of 17βHSD type 5 are expected to be effective for lung cancer.

As 17βHSD type 5 inhibitors, steroid derivatives (Patent Document 1) and NSAIDs (Non-steroidal Anti-Inflammatory Drugs) such as flufenamic acid, indomethacin and the like (Non-Patent Document 1), cinnamic acid derivatives (Non-Patent Document 2) and the like have been reported. Although the mechanism of action is different, a certain type of indazole derivative is known to be effective for BPH (Patent Document 2).

Meanwhile, a certain type of 3-(indol-1-yl) benzoic acid derivative (Patent Document 3), 3-(1H-benzimidazol-1-yl) benzoic acid derivative having a heat shock protein (HSP90)-inhibitory activity (Patent Document 4), or 3-(1H-benzimidazol-1-yl) benzoic acid derivative having a telomerase-inhibitory activity (Patent Document 5) is known to be effective for prostatic diseases such as prostate cancer. Further, a certain type of 3-(indol-1-yl) benzoic acid derivative (Patent Document 6), or a certain type of 3-(1H-benzimidazol-1-yl) benzoic acid derivative (Patent Documents 7 to 12) such as 3-[5-(trifluoromethyl)-1H-benzimidazol-1-yl] benzoic acid is known to be effective for central nervous system disorders such as Alzheimer's disease and dementia. Further, other 3-(1H-benzimidazol-1-yl) benzoic acid derivatives are also known to be effective for arteriosclerosis (Patent Document 13). In addition, 3-(1H-benzimidazol-1-yl) benzoic acid derivatives having an antiviral activity (Patent Document 14), a vascular endothelial growth factor (VEGF) receptor-antagonistic activity (Patent Document 15), or a platelet-derived growth factor (PDGF)-inhibitory activity (Non-Patent Documents 3 to 5), and 3-(indol-1-yl) benzoic acid derivatives (Patent Document 16) effective for pain or the like, are known. However, there is no report about the compounds effective for BPH, prostate cancer, or the like, as described in the present application.

[Patent Document 1] Pamphlet of International Publication No. WO99/046279
[Patent Document 2] Pamphlet of International Publication No. WO2004/064735
[Patent Document 3] Pamphlet of International Publication No. WO2003/057670
[Patent Document 4] US Patent Application Publication No. 2007/0105862 A I
[Patent Document 5] Pamphlet of International Publication No. WO2001/007020
[Patent Document 6] Pamphlet of International Publication No. WO2006/041874
[Patent Document 7] European Patent Application Publication No. 563001
[Patent Document 8] European Patent Application Publication No. 616807
[Patent Document 9] US Patent No. 5554630
[Patent Document 10] Pamphlet of International Publication No. WO96/033194
[Patent Document 11] Pamphlet of International Publication No. WO98/017651
[Patent Document 12] Pamphlet of International Publication No. WO98/034923
[Patent Document 13] JP-A-H 7-133224
[Patent Document 14] Pamphlet of International Publication No. WO97/033872
[Patent Document 15] JP-A-2002-193947
[Patent Document 16] Pamphlet of International Publication No. WO2008/006790
[Non Patent Document 1] "Cancer Research", 2004, Vol. 64, p. 1802-1810
[Non Patent Document 2] "Molecular and Cellular Endocrinology", 2006, Vol. 248, p. 233-235
[Non Patent Document 3] "The Journal of Medicinal Chemistry", 1998, Vol. 41, No. 27, p. 5457-5465
[Non Patent Document 4] "Bioorganic and Medicinal Chemistry", 2004, Vol. 12, No. 15, p 4009-4015
[Non Patent Document 5] "Bioorganic and Medicinal Chemistry", 2005, Vol. 13, No. 24, p. 6598-6608

### Disclosure of the Invention

### Problem that the Invention is to Solve

It is an object of the present invention to provide a compound useful as a pharmaceutical having a selective inhibitory activity against 17βHSD type 5, in particular as an agent for treating benign prostatic hyperplasia and/or prostate cancer.

### Means for Solving the Problem

As a result of intensive studies on compounds having a selective inhibitory activity against 17βHSD type 5, the present inventors have found that a compound, wherein the 1-position of the indole ring and/or the benzimidazole ring is substituted with a phenyl group having a carboxy group at the 3-position-, has a potent selective inhibitory activity against 17βHSD type 5 and can be an agent for treating and/or preventing diseases in which 17βHSD type 5 is involved such as benign prostatic hyperplasia and prostate cancer without accompanying adverse effects due to a decrease in testosterone. The invention has been completed based on these findings.

That is, the present invention relates to a pharmaceutical composition for preventing or treating a disease associated with 17βHSD type 5, comprising a compound of formula (I) or a pharmaceutically acceptable salt thereof as an active ingredient. 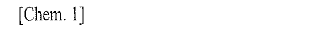 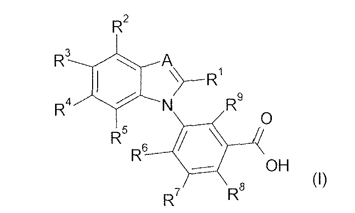 wherein in formula (I):
A represents C-R¹⁰ or N;
R¹ represents hydrogen, lower alkyl, halogeno lower alkyl, lower alkyl substituted with lower alkyl-O-, lower alkyl-O-, halogeno lower alkyl-O-, lower alkyl-O- substituted with lower alkyl-O-, cycloalkyl-L- which may be substituted, aryl-L- which may be substituted, or a heterocyclic group-L- which may be substituted;
L represents a bond, lower alkylene, lower alkenylene, O, lower alkylene-O, or O-lower alkylene;
R², R⁴, R⁵, R⁶, R⁷, R⁸, and R⁹, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
R³ represents hydrogen, halogen, cyano, nitro, halogeno lower alkyl, or lower alkyl-O-;
R¹⁰ represents hydrogen, aryl which may be substituted, or lower alkyl which may be substituted with hydroxy or lower alkyl-O-,
provided that,
when A is C-R¹⁰, R¹ represents hydrogen or lower alkyl;
when A is N, and R¹, R², R⁴, R⁵, R⁶, R⁷, R⁸, and R⁹ are hydrogen, R³ represents halogen, cyano, halogeno lower alkyl, or lower alkyl-O-;
when A is N, R¹ is methyl, and R², R³, R⁴, R⁵, R⁶, R⁷, and R⁹ are hydrogen, R⁸ is a group other than [(2E)-3-(2-naphthalenyl)-1-oxo-2-buten-1-yl]amino.

The meanings of the symbols are the same hereinafter.

Further, the present invention relates to a compound of formula (II) or a pharmaceutically acceptable salt thereof. 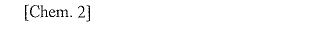 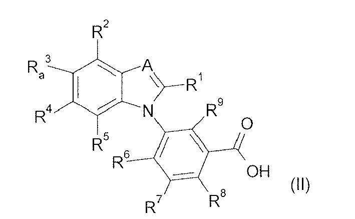 wherein in formula (II):
A represents C-R¹⁰ or N;
R¹ represents hydrogen, lower alkyl, halogeno lower alkyl, lower alkyl substituted with lower alkyl-O-, lower alkyl-O-, halogeno lower alkyl-O-, lower alkyl-O- substituted with lower alkyl-O-, cycloalkyl-L- which may be substituted, aryl-L- which may be substituted, or a heterocyclic group-L- which may be substituted;
L represents a bond, lower alkylene, lower alkenylene, O, lower alkylene-O, or O-lower alkylene;
R², R⁴, R⁵, R⁶, R⁷, R⁸, and R⁹, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
Rₐ³ represents hydrogen, halogen, cyano, halogeno lower alkyl, or lower alkyl-O-;
R¹⁰ represents hydrogen, aryl which may be substituted, or lower alkyl which may be substituted with hydroxy or lower alkyl-O-,
provided that,
when A is C-R¹⁰, R¹ represents hydrogen or lower alkyl, but when R¹, R², R⁵, R⁶, R⁷, R⁸, and R⁹ are hydrogen, R⁴ is chloro, and R¹⁰ is isobutyl, Rₐ³ is a group other than hydrogen;
when A is N, and R¹, R², R⁴, R⁵, R⁶, R⁷, R⁸, and R⁹ are hydrogen, Rₐ³ represents halogen, cyano, or lower alkyl-O-;
when A is N, R¹ is methyl, R², R⁴, R⁵, R⁶, R⁷, and R⁹ are hydrogen, and R⁸ is [(2E)-3-(2-naphthalenyl)-1-oxo-2-buten-1-yl]amino, Rₐ³ is a group other than hydrogen;
when A is N, and R¹, R², R⁵, R⁶, R⁷, R⁸, and R⁹ are hydrogen, and R⁴ is tert-butyl, Rₐ³ is a group other than hydrogen;
when A is N, R¹ is hydrogen or methyl, R², R⁶, and R⁷ are hydrogen, R⁴ and R⁸ are hydroxy, and R⁵ is carboxy, Rₐ³ is a group other than hydrogen.

The meanings of the symbols are the same hereinafter.

Further, the present invention relates to a pharmaceutical composition comprising the compound of formula (II) or a salt thereof, and a pharmaceutically acceptable excipient.

Further, the present invention relates to an agent for preventing and/or an agent for treating a disease associated with 17βHSD type 5, comprising the compound of formula (I) or a pharmaceutically acceptable salt thereof as an active ingredient.

Further, the present invention relates to use of the compound of formula (I) or a pharmaceutically acceptable salt thereof, for the manufacture of a pharmaceutical for treating and/or preventing a disease associated with 17βHSD type 5.

Further, the present invention relates to a method for treating and/or preventing a disease associated with 17βHSD type 5, comprising administering an effective amount of the compound of formula (I) or a pharmaceutically acceptable salt thereof to a patient.

Further, the present invention relates to an inhibitor of 17βHSD type 5, comprising the compound of formula (I) or a pharmaceutically acceptable salt thereof.

Further, the present invetion relates to a commercial package, comprising a pharmaceutical composition containing the compound of formula (I) or a pharmaceutically acceptable salt thereof; and a description that the compound of formula (I) or a pharmaceutically acceptable salt thereof is capable of being used or should be used for treating and/or preventing prostate cancer, benign prostatic hyperplasia, acne, seborrhea, hirsutism, baldness, alopecia, precocious puberty, adrenal hypertrophy, polycystic ovary syndrome, breast cancer, endometriosis, lung cancer or leiomyoma.

### Effects of the Invention

The compound of formula (I) inhibits 17βHSD type 5 selectively. Accordingly, the compound of formula (I) is useful as an agent for preventing and/or treating diseases associated with 17βHSD type 5, for example, diseases associated with androgen, since androgen synthesis is suppressed by the inhibition of 17βHSD type 5. Examples of the androgen-associated disease include prostate cancer, benign prostatic hyperplasia, acne, seborrhea, hirsutism, baldness, alopecia, precocious puberty, adrenal hypertrophy, polycystic ovary syndrome, breast cancer, endometriosis, leiomyoma and the like. In addition, since AKR1C3 (another name for 17βHSD type 5), which is a subtype of aldo-keto reductase (AKR), metabolizes Polycyclic Aromatic Hydrocarbon (PAH) to generate reactive oxygen species (ROS) and since single nucleotide polymorphism (SNP) of AKR1C3 gene relating to oxidation stress correlates to the risk of lung cancer, lung cancer is also exemplified as a disease associated with 17βHSD type 5. The compound of formula (I) is therefore useful as an agent for treating and/or an agent for preventing these diseases.

In addition, since 17βHSD type 5 is considered to be responsible for intracrine androgen synthesis in the prostate, selective inhibitors of 17βHSD type 5 are expected to suppress intracrine androgen synthesis in the prostate selectively. Accordingly, the compound of formula (I) is particularly useful as an agent for treating and/or preventing diseases associated with androgen in the prostate, that is, prostate cancer and benign prostatic hyperplasia.

Further, since the compound of formula (I) does not influence the concentrations of testosterone in the blood, the compound may be an agent for treating and/or preventing benign prostatic hyperplasia and prostate cancer without adverse effects such as sexual dysfunction due to supression of the blood testosterone concentration, and the like.

### Best mode for carrying out the invention

Hereinafter, the present invention will be described in detail.
Hereinafther, the compound of formula (I) and/or formula (II) will be described in detail. Since the compound of formula (II) is included in the compound of formula (I) and is one of the preferred embodiments of the compound of formula (I), the following describe mainly the compound of formula (I).

Various preferred examples of the definitions included in the scope of the present invention, in the description above and below in the present specification will be described in detail below.
The term "lower" means a group containing 1 to 10 carbon atoms (hereinafter, referred to also as "C₁₋₁₀"), unless otherwise particularly noted.

The "lower alkyl" means linear or branched alkyl containing I to 10 carbon atoms, preferably C₁₋₆ alkyl, and for example, methyl, ethyl, propyl, isopropyl, butyl, isobutyl, tert-butyl, pentyl, hexyl group and the like.
The "lower alkylene" means a divalent group formed by removing a hydrogen atom from the "lower alkyl".

The "lower alkenyl" means an unsaturated linear or branched noncyclic hydrocarbon containing 2 to 10 carbon atoms having at least one double bond. The number of carbon atoms is preferably 2 to 6. For example, vinyl, propenyl, butenyl, pentenyl, hexenyl and the like are included.
The "lower alkenylene" means a divalent group formed by removing a hydrogen atom from the "lower alkenyl".

The "halogen" includes F, Cl, Br, or I and is preferably F, Cl, or Br.

The "halogeno lower alkyl" is lower alkyl substituted with one or more halogens. In another embodiment, it means lower alkyl substituted with 1 to 10 halogens. For example, fluoromethyl, chloromethyl, difluoromethyl, dichloromethyl, dibromomethyl, trifluoromethyl, trichloromethyl, fluoroethyl, chloroethyl, 2,2,2-trifluoroethyl, 2,2,2-trichloroethyl, 2,2,3,3,3-pentafluoropropyl, fluoropropyl, fluorobutyl, fluorohexyl and the like are included.

The "cycloalkyl" is a saturated hydrocarbon ring group containing 3 to 10 carbon atoms and optionally having a bridge. For example, cyclopropyl, cyclobutyl, cyclopentyl, cyclohexyl, cycloheptyl, norbornyl, adamantyl group and the like are included.

The "aryl" is a C₆₋₁₄ monocyclic to tricyclic aromatic hydrocarbon ring group which contains a ring group condensed with C₅₋₈ cycloalkene at the double bond site thereof. For example, phenyl, naphthyl, tetrahydronaphthalenyl, indenyl, fluorenyl group, and the like are included. In another embodiment, it is phenyl, naphthyl, or anthryl. In yet another embodiment, it is phenyl.

The "heterocyclic" group means 3 to 6-membered monocyclic heteroaromatic ring groups having preferably I to 4 nitrogen atoms, for example, pyrrolyl, imidazolyl, pyrazolyl, pyridyl, pyrimidinyl, pyrazinyl, pyridazinyl, triazolyl (for example, 4H-1,2,4-triazolyl, 1H-1,2,3-triazolyl, 2H-1,2,3-triazolyl and the like), tetrazolyl (for example, 1H-tetrazolyl, 2H-tetrazolyl and the like) and the like; condensed heteroaromatic ring groups having 1 to 5 nitrogen atoms, for example, indolyl, isoindolyl, indolizinyl, benzimidazolyl, quinolyl, isoquinolyl, indazolyl, benzotriazolyl, tetrazolopyridazinyl (for example, tetrazolo[1,5-b]pyridazinyl and the like), quinoxalinyl, imidazopyridyl (for example, imidazo[1,2-a]pyridyl and the like) and others; 3 to 6-membered monocyclic heteroaromatic ring groups having one oxygen atom, for example, pyranyl, furyl and the like; 3 to 6-membered monocyclic heteroaromatic ring groups having 1 to 2 sulfur atoms, for example, thienyl and the like; 3 to 6-membered monocyclic heteroaromatic ring groups having 1 to 2 oxygen atoms and 1 to 3 nitrogen atoms, for example, oxazolyl, isooxazolyl, oxadiazolyl (for example, 1,2,4-oxadiazolyl, 1,3,4-oxadiazolyl, 1,2,5-oxadiazolyl and the like) and others; condensed heteroaromatic ring groups having 1 to 2 oxygen atoms and 1 to 3 nitrogen atoms, for example, benzofurazanyl, benzoxazolyl, benzoxadiazolyl and the like; 3 to 6-membered monocyclic heteroaromatic ring groups having 1 to 2 sulfur atoms and 1 to 3 nitrogen atoms, for example, thiazolyl, thiadiazolyl (for example, 1,2,4-thiadiazolyl, 1,3,4-thiadiazolyl, 1,2,5-thiadiazolyl and the like) and others; condensed heteroaromatic ring groups having 1 to 2 sulfur atoms and 1 to 3 nitrogen atoms, for example, benzothiazolyl, benzothiadiazolyl, imidazothiazolyl (for example, imidazo[2,1-b][1,3]thiazolyl and the like) and others; condensed heteroaromatic ring groups having 1 to 2 oxygen atoms, for example, benzofuranyl, dibenzo[b,d]furanyl and the like; condensed heteroaromatic ring groups having I to 2 sulfur atoms, for example, benzothienyl and the like; and others.

The "heterocyclic" group further means a 3 to 10-membered saturated or unsaturated ring group containing 1 to 4 hetero atoms selected from O, N and S, and preferable examples thereof include 3 to 7-membered saturated heteromonocyclic groups having I to 4 nitrogen atoms, for example, pyrrolidinyl, imidazolidinyl, piperidyl, piperazinyl, and the like; 3 to 10-membered saturated or unsaturated bicyclic heterocyclic groups having 1 to 4 nitrogen atoms, for example, quinuclidinyl and the like; 3 to 6-membered saturated heteromonocyclic groups having one oxygen atom, for example, 1H-tetrahydropyranyl, tetrahydrofuranyl and the like; 3 to 6-membered saturated or unsaturated heteromonocyclic groups having 1 to 2 oxygen atoms and 1 to 3 nitrogen atoms, for example, morpholinyl, oxazolinyl (for example, 2-oxazolinyl and the like) and others; 3 to 6-membered saturated or unsaturated heteromonocyclic groups having 1 to 2 sulfur atoms and 1 to 3 nitrogen atoms, for example, thiazolidinyl and others; and the like.

The "heteroaryl" means a group included in the "heterocyclic" group and having an aromaticity.

The "cyano lower alkyl" is lower alkyl substituted with one or more cyano groups. In another embodiment, it means lower alkyl substituted with I to 3 cyano groups. For example, cyanomethyl, cyanoethyl, 2,3-dicyanopropyl, and the like are included.

The "acyl group" include, for example, carboxy; esterified carboxy; carbamoyl substituted with lower alkyl, aryl, aryl-lower alkylene, arylsulfonyl, sulfonyl substituted with heterocyclic group, lower alkylsulfonyl or heterocyclic group; substituted or unsubstituted arylsulfonyl; sulfonyl substituted with a heterocyclic group; lower alkylsulfonyl; cycloalkylcarbonyl; lower alkyl-CO-; HCO-; substituted or unsubstituted aryl-CO-; carbonyl substituted with a heterocyclic group, and the like.

The esterified carboxy group include substituted or unsubstituted lower alkyl-O-C(=O)- (for example, methoxycarbonyl, ethoxycarbonyl, propoxycarbonyl, butoxycarbonyl, tert-butoxycarbonyl, hexyloxycarbonyl, 2-iodoethoxycarbonyl, 2,2,2-trichloroethoxycarbonyl and the like), substituted or unsubstituted aryl-O-C(=O)- (for example, phenoxycarbonyl, 4-nitrophenoxycarbonyl, 2-naphthyloxycarbonyl and the like), substituted or unsubstituted aryl-lower alkylene-O-C(=O)- (for example, benzyloxycarbonyl, phenethyloxycarbonyl, benzhydryloxycarbonyl, 4-nitrobenzyloxycarbonyl and the like) and others.

The carbamoyl group substituted with lower alkyl include methylcarbamoyl, ethylcarbamoyl, propylcarbamoyl, dimethylcarbamoyl, diethylcarbamoyl, methylethylcarbamoyl and the like.

The carbamoyl group substituted with aryl include phenylcarbamoyl, naphthylcarbamoyl, phenylcarbamoyl substituted with lower alkyl (for example, tolylcarbamoyl, xylylcarbamoyl and the like) and others.

The carbamoyl group substituted with aryl-lower alkylene include benzylcarbamoyl, phenethylcarbamoyl, phenylpropylcarbamoyl and the like.

The carbamoyl group substituted with arylsulfonyl include phenylsulfonylcarbamoyl, tolylsulfonylcarbamoyl and the like.

The carbamoyl substituted with sulfonyl substituted with a heterocyclic group include pyridylsulfonylcarbamoyl and the like.

The carbamoyl group subsituted with a lower alkylsulfonyl include methylsulfonylcarbamoyl, ethylsulfonylcarbamoyl and the like.

The substituted or unsubstituted arylsulfonyl group include phenylsulfonyl, tolylsulfonyl, halophenylsulfonyl (for example, fluorophenylsulfonyl and the like) and others.

The sulfonyl group substituted with a heterocycle include pyrrolidinylsulfonyl and the like.

The lower alkylsulfonyl group include methylsulfonyl, ethylsulfonyl and the like.

The cycloalkylcarbonyl group include, for example, cyclopropylcarbonyl, cyclobutylcarbonyl, cyclopentylcarbonyl or cyclohexylcarbonyl and the like.

The lower alkyl-CO- group include acetyl, propionyl, butyryl, isobutyryl, valeryl, isovaleryl, pivaloyl, hexanoyl and the like.

The substituted or unsubstituted aryl-CO- group include benzoyl, naphtoyl, toluoyl, di(tert-butyl)benzoyl, halogeno lower alkyl-O-benzoyl (for example, trifluoromethoxybenzoyl and the like) and others.

The heterocyclic group moiety of the "carbonyl substituted with a heterocyclic group" means the "heterocyclic" group described above. For example, pyridylcarbonyl and the like are included.

The "mono-lower alkylamino" means an amino group substituted with a "lower alkyl" group described above. For example, methylamino, ethylamino, propylamino, isopropylamino, butylamino, isobutylamino, pentylamino, hexylamino, and the like are included.

The "di-lower alkylamino" means an amino group substituted with two same or different "lower alkyl" described above. For example, dimethylamino, diethylamino, dipropylamino, diisopropylamino, dibutylamino, diisobutylamino, dipentylamino, dihexylamino, ethylmethylamino, methylpropylamino, butylmethylamino, ethylpropylamino, butylethylamino and the like are included.

The "which may be substituted" means unsubstituted or substituted with 1 to 5 substituents. The "substituted" means that having 1 to 5 substituents. Further, if there are multiple substituents, the substituents may be the same or different from each other.

The substituents for the "cycloalkyl which may be substituted", "aryl which may be substituted" or "heterocyclic group which may be substituted" in of R¹ and R¹⁰ include, for example, lower alkyl, halogen, cyano, lower alkenyl, cycloalkyl, halogeno lower alkyl, lower alkyl-O-, cyano lower alkyl-O-, halogeno lower alkyl-O-, aryl, heteroaryl, aryl-O-, heteroaryl-O-, aryl-lower alkylene-, acyl, acyl-O-, heteroaryl-lower alkylene-O-, lower alkylsulfanyl, nitro, amino, hydroxy, sulfanyl, mono-lower alkyl amino, di-lower alkylamino, acylamino, and arylamino group. In another embodiment, a group selected from lower alkyl, halogen, cyano, lower alkenyl, halogeno lower alkyl, lower alkyl-O-, cyano lower alkyl-O-, halogeno lower alkyl-O-, and lower alkyl-CO-, is exemplified. In yet another embodiment, a group selected from lower alkyl, halogen, halogeno lower alkyl, lower alkyl-O-, and lower alkyl-CO- is exemplified.

Although "cycloalkyl", "phenyl", "cyclohexyl" and the like are described as monovalent groups in the present specification for convenience, they may be multivalent groups of divalent or higher valency according to their structures. The present invention encompasses these structures. Specific embodiments of the divalent group correspond to those having the suffix of the above ring groups converted into diyl in accordance with the Nomenclature of Organic Chemistry. For example, a divalent group corresponding to a phenyl group that is a monovalent group is a phenylene. A divalent group corresponding to a benzothiazolyl group that is a monovalent group is benzothiazolediyl.

The "selective inhibitor of 17βHSD type 5 (AKR1C3)" means an inhibitor which exhibits 3-fold or more, preferably 10-fold or more, and more preferably 100-fold or more value for an inhibitory activity against 17βHSD type 3 and AKR1C2 compared to an inhibitory activity against human 17βHSD type5(AKR1C3), in terms of IC₅₀ value.

An embodiment of the compound of formula (I) as an active ingredient of the present invention will be described below.
(1) A compound of formula (II).
(2) The compound wherein A is C-R¹⁰
(3) The compound wherein A is N.
(4) The compound wherein R¹ is hydrogen.
(5) The compound wherein R¹ is lower alkyl.
(6) The compound wherein R¹ is halogeno lower alkyl.
(7) The compound wherein R¹ is lower alkyl substituted with lower alkyl-O-.
(8) The compound wherein R¹ is lower alkyl substituted with phenyl wherein the phenyl may be substituted with a group selected from lower alkyl, halogen, halogeno lower alkyl, and lower alkyl-O-.
(9) The compound wherein R¹ is lower alkyl substituted with phenyl-O-, wherein the phenyl may be substituted with a group selected from lower alkyl, halogen, halogeno lower alkyl, and lower alkyl-O-.
(10) The compound wherein R¹ is lower alkyl-O- which may be substituted with phenyl.
(11) The compound wherein R¹ is cycloalkyl.
(12) The compound wherein R¹ is phenyl which may be substituted with a group selected from lower alkyl, halogen, and lower alkyl-O-.
(13) The compound wherein R² is hydrogen.
(14) The compound wherein R⁴ is hydrogen.
(15) The compound wherein R⁵ is hydrogen.
(16) The compound wherein R⁶ is hydrogen.
(17) The compound wherein R⁷ is hydrogen.
(18) The compound wherein R⁸ is hydrogen.
(19) The compound wherein R⁹ is hydrogen.
(20) The compound wherein R², R⁴, R⁵, R⁶, R⁷, R⁸, and R⁹, which are the same or different from each other, are hydrogen or halogen.
(21) The compound wherein R³ or Rₐ³ is halogeno lower alkyl or cyano.
(22) The compound wherein R³ or Rₐ³ is halogeno lower alkyl.
(23) The compound wherein R³ or Rₐ³ is cyano.
(24) The compound wherein R³ or Rₐ³ is trifluoromethyl.
(25) The compound which is a combination of any two or more of (1) to (24) above.

Specific examples of the compound of the above (25) include the following compounds.
(26) A compound of formula (II) wherein A is C-R¹⁰.
(27) The compound described in (26), wherein R¹ is hydrogen, and R², R⁴, R⁵, R⁶, R⁷, R⁸, and R⁹, which are the same or different from each other, are hydrogen or halogen.
(28) The compound described in (27), wherein Rₐ³ is halogeno lower alkyl, or cyano.
(29) A compound of formula (II) wherein A is N.
(30) The compound described in (29), wherein R², R⁴, R⁵, R⁶, R⁷, R⁸, and R⁹, which are the same or different from each other, are hydrogen or halogen.
(31) The compound described in (30), wherein Rₐ³ is halogeno lower alkyl, or cyano.
(32) The compound described in (31) above, wherein Rₐ³ is trifluoromethyl.
(33) The compound described in any of (29) to (32), wherein R¹ represents hydrogen; lower alkyl; halogeno lower alkyl; lower alkyl substituted with lower alkyl-O-, phenyl or phenyl-O-; phenyl which may be substituted with lower alkyl, halogen, or lower alkyl-O-; or cycloalkyl, wherein the phenyl moiety in the lower alkyl substituted with phenyl or phenyl-O- may be substituted with lower alkyl, halogen, halogeno lower alkyl, or lower alkyl-O-.
(34) The compound described in (32), wherein R¹ is lower alkyl substituted with hydrogen, lower alkyl, halogeno lower alkyl, or lower alkyl-O-.
(35) The compound described in (32), wherein R¹ is lower alkyl-O- which may be substituted with phenyl.
(36) The compound described in (32), wherein R¹ is cycloalkyl.
(37) The compound described in (32), wherein R¹ is phenyl which may be substituted with a group selected from lower alkyl, halogen, and lower alkyl-O-.
(38) The compound described in (32), wherein R¹ is lower alkyl which is substituted with phenyl or lower alkyl substituted with phenyl-O-, wherein the said phenyl may be substituted with a group selected from lower alkyl, halogen, halogeno lower alkyl, and lower alkyl-O-.

Specific compounds included in the present invention are the following compounds.
3-(5-cyano-1H-indol-1-yl) benzoic acid,
3-[3-phenyl-5-(trifluoromethyl)-1H-indol-1-yl] benzoic acid,
3-[3-(2-methoxyethyl)-5-(trifluoromethyl)-1H-indol-1-yl] benzoic acid,
3-[5-(tritluorometlryl)-1H-benzimidazol-1-yl] benzoic acid,
3-[2-methyl-5-(trifluoromethyl)-1H-benzimidazol-1-yl] benzoic acid,
3-[2-etlryl-5-(trifluoromethyl)-1H-benzimidazol-1-yl] benzoic acid,
3-[2-propyl-5-(trifluoromethyl)-1H-benzimidazol-1-yl] benzoic acid,
3-[2,5-bis(trifluoromethyl)-1H-benzimidazol-1-yl] benzoic acid,
3-[2-phenoxymethyl-5-(trifluoromethyl)-1H-benzimidazol-1-yl] benzoic acid,
3-[2-methoxy-5-(trifluoromethyl)-1H-benzimidazol-1-yl] benzoic acid,
3-[2-cyclopropyl-5-(trifluoromethyl)-1H-benzimidazol-1-yl] benzoic acid,
3-[2-cyclohexyl-5-(trifluoromethyl)-1H-benzimidazol-1-yl] benzoic acid,
3-[2-phenyl-5-(trifluoromethyl)-1H-benzimidazol-1-yl] benzoic acid,
3-[2-(3-methylphenyl)-5-(trifluoromethyl)-1H-benzimidazol-1-yl] benzoic acid,
3-[2-benzyl-5-(trifluoromethyl)-1H-benzimidazol-1-yl] benzoic acid,
3-[2-(3-fluorobenzyl)-5-(trifluoromethyl)-1H-benzimidazol-1-yl] benzoic acid,
3-[2-(4-fluorobenzyl)-5-(trifluoromethyl)-1H-benzimidazol-1-yl] benzoic acid,
3-[2-(4-chlorobenzyl)-5-(trifluoromethyl)-1H-benzimidazol-1-yl] benzoic acid, and
3-[2-(benzyloxy)-5-(trifluoromethyl)-1H-benzimidazol-1-yl] benzoic acid.

The compound of formula (I) may in some cases exist in the form of tautomers or geometrical isomers, depending on the kind of substituents. In the present specification, the compound of formula (I) may be described only in one form of the isomers, but the present invention includes other isomers as well as isolated forms or mixtures thereof.
Further, the compound of formula (I) may have asymmetric carbon atoms or axial asymmetries in some cases, and correspondingly, it may exist in the form of optical isomers. The present invention also includes isolates or mixtures of optical isomers of the compound of formula (I).

The compound of formula (I) may be converted into a salt thereof by an general method. The salt of the compound of formula (I) is a pharmaceutically acceptable salt. Examples of the salt include metal salts such as alkali metal salts (for example, sodium salt, potassium salt, and the like) or alkaline earth metal salts (for example, calcium salt, magnesium salt, and the like), ammonium salts, organic base salts (for example, trimethylamine salt, triethylamine salt, pyridine salt, picoline salt, dicyclohexylamine salt, and the like), organic acid salts (for example, acetate, malonate, tartrate, methanesulfonate, benzenesulfonate, formate, toluenesulfonate, trifluoroacetate, and the like), inorganic acid salts (for example, hydrochloride, hydrobromide, sulfate, phosphate, and the like), amino acid salts (for example, salt with arginine, aspartic acid, glutamic acid, and the like) and others. Accordingly, the present invention encompasses all of the compounds of formula (I) and pharmaceutically acceptable salts thereof.

The present invention also includes various hydrates or solvates, and polymorphisms of the compound of formula (I) and a pharmaceutically acceptable salt thereof. Further, the present inventnion include compounds labeled with various radioactive or non-radioactive isotopes.

Further, a pharmacologically acceptable prodrug of the compound of formula (I) is also included in the present invention. The pharmacologically acceptable prodrug is a compound having a group which can be converted into an amino group, a hydroxyl group, a carboxyl group or the like by solvolysis or under a physiological condition. Examples of the group for forming a prodrug include those as described, for example, in Prog. Med., 5, 2157-2161 (1985) or "Pharmaceutical Research and Development" (Hirokawa Publishing Company, 1990), Vol. 7, "Drug Design", 163-198.

### (General Production Process)

The compound of formula (I) as an active ingredient of the present invention can be produced by utilizing the characteristics based on the types of basic skeleton or substituents and by applying various known synthetic methods. It is sometimes effective, in terms of production techniques, that the functional group is protected by an appropriate protecting group or replaced by a group that can be easily converted into the functional group in the stage of a starting material to intermediate, depending on the type of the functional group during the production. Examples of such functional groups include an amino group, a hydroxy group, a carboxyl group, and the like, and examples of such protecting groups include protecting groups described, for example, in "Protective Groups in Organic Synthesis", (3rd eddition, 1999), edited by T.W. Greene and P.G.M. Wuts. These protecting groups may be appropriately selected and used depending on the reaction conditions. According to such a method, a desired compound can be obtained by introducing the protecting group and carrying out the reaction, and then removing the protecting group, or converting the protecting group into a desired group, if desired.

### (Production Process 1)

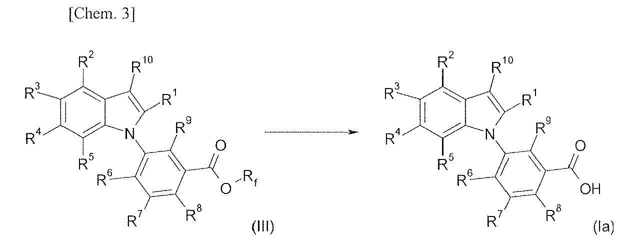

Production Process I is a method in which a compound of formula (Ia) is produced by subjecting a compound of formula (III), (wherein in the formula, R_{f} represents lower alkyl), to hydrolysis.

The hydrolysis may be carried out under either of acidic conditions or basic conditions. The hydrolysis is preferably carried out under basic conditions. The base to be used is preferably an inorganic base such as sodium hydroxide, potassium hydroxide, sodium carbonate, potassium carbonate, cesium carbonate, or the like.

The solvent used is not particularly limited, as long as the solvent does not inhibit the reaction and dissolves the starting materials to a certain level, and examples thereof include alcohols such as methanol, ethanol, and 2-propanol; ethers such as diethyl ether, diisopropyl ether, tetrahydrofuran, dioxane, dimethoxyethane, diethylene glycol dimethyl ether, tert-butyl methyl ether or cyclopentyl methyl ether; or water, or a mixture thereof. Preferred is a mixed solvent of tetrahydrofuran and water.

The reaction temperature of this reaction is generally 0°C to 100°C, preferably 0°C to 50°C, varying depending on the starting compounds, reagents and the like.
The reaction time of this reaction is generally 5 minutes to 24 hours, preferably 10 minutes to 12 hours, varying depending on the starting compounds, reagents, the reaction temperature and the like.

### (Production Process 2)

### (Production Process 2-1)

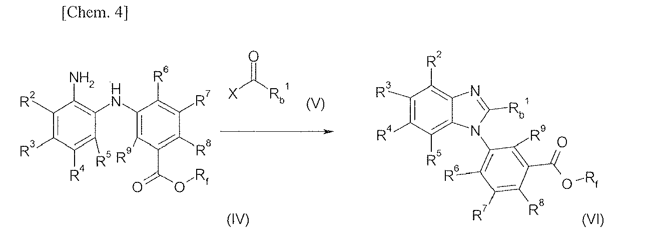

Production Process 2-1 is a method in which a compound of formula (VI) (wherein in the formula, R_{b}¹ and R_{f} are as defined below) is obtained by reacting a compound of formula (IV) (wherein in the formula, R_{f} represents lower alkyl) and a compound of formula (V) (wherein in the formula, R_{b}¹ represents lower alkyl, halogeno lower alkyl, lower alkyl substituted with lower alkyl-O-, cycloalkyl-Lₐ- which may be substituted, aryl-Lₐ- which may be substituted, or a heterocyclic group-Lₐ- which may be substituted; Lₐ is a bond, lower alkylene, or lower alkenylene; and X represents a leaving group.)

### (Production Process 2-2)

Production Process 2-2 is a method in which a compound of formula (Ib) (wherein in the formula, R_{b}¹ is as defined above) is obtained by hydrolysis of the resulting compound of formula (VI).

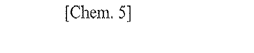

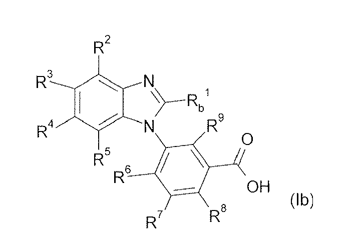

The solvent used in Production Process 2-1 is not particularly limited, as long as the solvent does not inhibit the reaction and dissolves the starting materials to a certain level, and examples thereof include aromatic hydrocarbons such as benzene, toluene or xylene; halogenated hydrocarbons such as dichloromethane, chloroform, carbon tetrachloride, 1,2-dichloroethane, chlorobenzene or dichlorobenzene; ethers such as diethyl ether, diisopropyl ether, tetrahydrofuran, dioxane, dimethoxyethane, diethylene glycol dimethyl ether, tert-butyl methyl ether or cyclopentyl methyl ether; nitro compounds such as nitromethane; nitrides such as acetonitrile or isobutyronitrile; amides such as formamide, N,N-dimethylformamide, N,N-dimcthylacetamide or N-methyl-2-pyrrolidinone; sulfoxides such as dimethyl sulfoxides; sulfones such as sulfolane; alcohols such as methanol, ethanol, or 2-propanol; and a mixture thereof. Preferred are ethers, particularly tetrahydrofuran.

Examples of the leaving group X include a halogen atom, a lower alkoxy group, a phenoxy group, an imidazolyl group, and the like.

The reaction temperature of Production Process 2-1 is generally 0°C to 100°C, preferably 20°C to 100°C, varying depending on the starting compounds, reagents and the like.
The reaction time of Production Process 2-1 is generally 5 minutes to 24 hours, preferably 10 minutes to 12 hours, varying depending on the starting compounds, reagents, the reaction temperature and the like.

Production Process 2-2 may be carried out under the same conditions as hydrolysis of Production Process 1.

Further, the compound of formula (Ib) may be obtained by changing the reaction order of Production Process 2-1 and Production Process 2-2, that is by first hydrolyzing the compound of formula (IV) and then reacting with the compound of formula (V). In this case, respective reaction conditions are as set forth above.

### (Production Process 3)

### (Production Process 3-1)

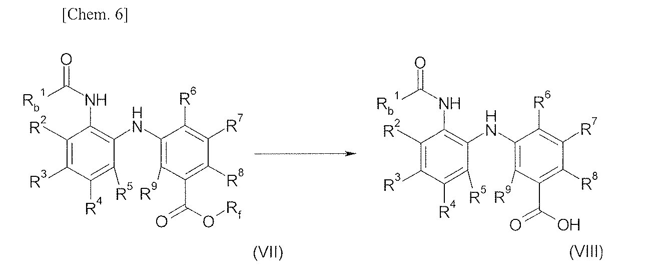

In Production Process 2-1, the compound of formula (VII) (wherein in the formula, R_{b}¹ and R_{f} are as defined above) may be isolated, but not the compound of formula (VI). Production Process 3-1 is a method in which a compound of formula (VIII) (wherein in the formula, R_{b}¹ is as defined above) is obtained by hydrolyzing a compound of formula (VII).

### (Production Process 3-2)

Production Process 3-2 is a method in which a compound of formula (Ib) is obtained by heating the resulting compound of formula (VIII) under acidic conditions.

Production Process 3-1 may be carried out under the same conditions as hydrolysis of Production Process 1.

The solvent used in Production Process 3-2 may suitably be an acidic solvent, for example, acetic acid, trifluoroacetic acid, hydrochloric acid, or the like.
The reaction temperature of Production Process 3-2 is generally 0°C to 100°C, preferably 20°C to 100°C, varying depending on the starting compounds, reagents and the like.

### (Production Process 4)

### (Production Process 4-1)

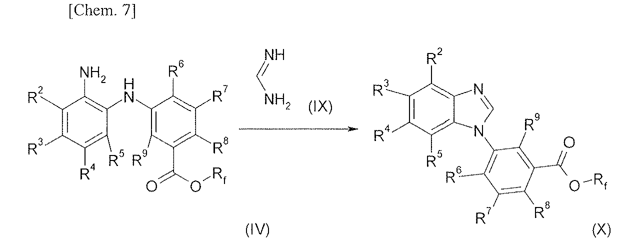

Production Process 4-1 is a method in which a compound of formula (X) (wherein in the formula, R_{f} is as defined above) is obtained by reacting a compound of formula (IV) with a compound of formula (IX) or an acid addition salt of (IX).

### (Production Process 4-2)

Production Process 4-2 is a method in which a compound of formula (Ic) is obtained by hydrolizing the resulting compound of formula (X).

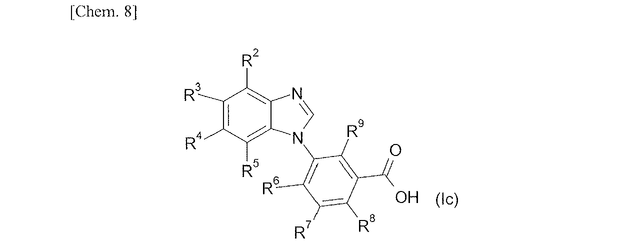

The solvent used in Production Process 4-1 is not particularly limited, as long as the solvent does not inhibit the reaction and dissolves the starting materials to a certain level, and examples thereof include aromatic hydrocarbons such as benzene, toluene or xylene; halogenated hydrocarbons such as dichloromethane, chloroform, carbon tetrachloride, 1,2-dichloroethane, chlorobenzene or dichlorobenzene; ethers such as diethyl ether, diisopropyl ether, tetrahydrofuran, dioxane, dimethoxyethane, diethylene glycol dimethyl ether, tert-butyl methyl ether or cyclopentyl methyl ether; nitro compounds such as nitromethane; nitriles such as acetonitrile or isobutyronitrile; amides such as formamide, N,N-dimethylformamide, N,N-dimethylacetamide or N-methyl-2-pyrrolidinone; sulfoxides such as dimethyl sulfoxide; sulfones such as sulfolane; alcohols such as methanol, ethanol, or 2-propanol; and a mixture thereof. Preferred are alcohols, particularly ethanol.

The reaction temperature of Production Process 4-1 is generally 0°C to 100°C, preferably 20°C to 100°C, varying depending on the starting compounds, reagents and the like.
The reaction time of Production Process 4-1 is generally 5 minutes to 24 hours, preferably 10 minutes to 12 hours, varying depending on the starting compounds, reagents, the reaction temperature and the like.

Production Process 4-2 may be carried out under the same conditions as hydrolysis of Production Process 1.

### (Production Process 5)

### (Production Process 5-1)

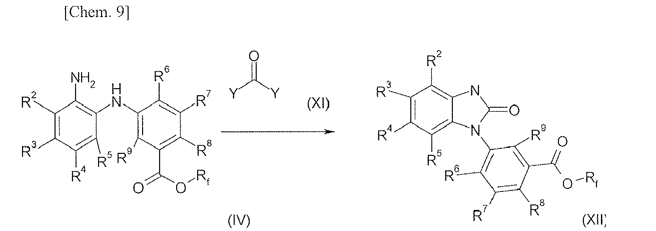

Production Process 5-1 is a method in which a compound of formula (XII) (wherein in the formula, R_{f} is as defined below) is obtained by reacting a compound of formula (IV) (wherein in the formula, R_{f} represents lower alkyl) with a compound of formula (XI).

### (Production Process 5-2)

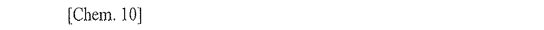

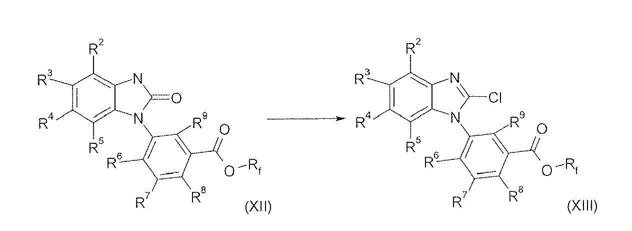

Production Process 5-2 is a method in which a compound of formula (XIII) (wherein in the formula, I_{f} is as defined above) is obtained by subjecting the resulting compound of formula (XII) to halogenation.

### (Production Process 5-3)

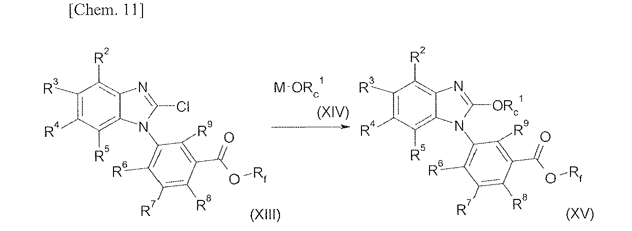

Production Process 5-3 is a method in which a compound of formula (XV) (wherein in the formula, R_{c}¹ and R_{f} are as defined below) is obtained by reacting the resulting compound of formula (XIII) with a compound of formula (XIV) (wherein in the formula, R_{c}¹ represents lower alkyl, halogeno lower alkyl, lower alkyl substituted with lower alkyl-O-, cycloalkyl which may be substituted, aryl which may be substituted, or a heterocyclic group which may be substituted; and M represents a metal.)

### (Production Process 5-4)

Production Process 5-4 is a method in which a compound of formula (Id) is obtained by hydrolyzing the resulting compound of formula (XV).

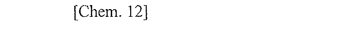

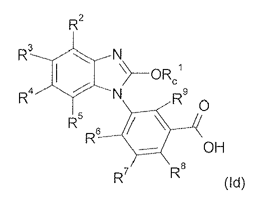

Production Process 5-1 may be carried out under the same conditions as in Production Process 2-1. Further, it may be advantageous in some cases for the smooth progress of the reaction to carry out the reaction in the presence of an organic base such as triethylamine, N,N-diisopropylethylamine, or N-methylmorpholine, or an inorganic base such as potassium carbonate, sodium carbonate, or potassium hydroxide.

Examples of the leaving group Y include a halogen atom, a lower alkoxy group, a phenoxy group, an imidazolyl group, and the like. Preferred is an imidazolyl group.

Production Process 5-2 may be carried out by dissolving the resulting compound of formula (XII) in phosphorus oxychloride or the like, and then adding phosphorus pentachloride or the like if necessary, followed by heating.

The reaction temperature of Production Process 5-2 is generally 0°C to 120°C, preferably 20°C to 100°C, varying depending on the starting compounds, reagents and the like.
The reaction time of Production Process 5-2 is generally 5 minutes to 24 hours, preferably 10 minutes to 20 hours, varying depending on the starting compounds, reagents, the reaction temperature and the like.

The solvent used in Production Process 5-3 is not particularly limited, as long as the solvent does not inhibit the reaction and dissolves the starting materials to a certain level, and examples thereof include aromatic hydrocarbons such as benzene, toluene or xylene; ethers such as diethyl ether, diisopropyl ether, tetrahydrofuran, dioxane, dimethoxyethane, diethylene glycol dimethyl ether, tert-butyl methyl ether or cyclopentyl methyl ether; amides such as formamide, N,N-dimethylformamide, N,N-dimethylacetamide or N-methyl-2-pyrrolidinone; sulfoxides such as dimethyl sulfoxide; sulfones such as sulfolane; alcohols such as methanol, ethanol, or 2-propanol; and a mixture thereof. Preferred are alcohols, N,N-dimethylformamide, or dimethyl sulfoxide.

Examples of the metal M include alkali metals such as lithium, sodium, and potassium, alkaline earth metals such as magnesium, and calcium, or the like.

The reaction temperature of Production Process 5-3 is generally 0°C to 100°C, preferably 20°C to 100°C, varying depending on the starting compounds, reagents and the like.
The reaction time of Production Process 5-3 is generally 5 minutes to 24 hours, preferably 10 minutes to 20 hours, varying depending on the starting compounds, reagents, the reaction temperature and the like.

Production Process 5-4 may be carried out under the same conditions as hydrolysis of Production Process 1.

Further, the compound of formula (Id) may be obtained by changing the reaction order of Production Process 5-3 and Production Process 5-4, that is, by first hydrolyzing the compound of formula (XIII) and then reacting with the compound of formula (XIV). In this case, respective reaction conditions are as set forth above.

In addition, other compounds of formula (I) may be obtained by applying the methods known to the person skilled in the art, such as ordinary alkylation, esterification, amidation, acylation, and reduction (including reduction of aryl and/or heteroaryl, dehalogenation, and the like), to the compound of formula (I) or a pharmaceutically acceptable salt thereof.

The compound of formula (I) having various functional group(s) can also be produced by applying methods well known to the person skilled in the art or known production processes, or variations thereof. For example, a desired compound can be produced by subjecting the compound obtained by the production process described above to a substituent-modification reaction. Representative reactions are shown below.

### (1) Alkylation

Among the compound of formula (I), the compound having a lower alkoxy group or a lower alkylamino group can be produced by subjecting a compound having a hydroxy group or an amino group to an alkylation reaction. For example, the reaction can be carried out by the methods described in "The fourth edition of Courses in Experimental Chemistry (Vol. 20)" edited by The Chemical Society of Japan, Maruzen, 1992 and "The fifth edition of Courses in Experimental Chemistry (Vol. 14)" edited by The Chemical Society of Japan, Maruzen, 2005; or "Compendium of Organic Synthetic Methods", Vols. 1 to 3, or the like.

### (2) Reduction

Among the compound of formula (I), the compound having a saturated cycloalkyl group or a saturated heterocyclic group can also be produced by reduction of aryl and/or heteroaryl. The reaction can be carried out by selecting and using the reaction conditions described, for example, in "The fourth edition of Courses in Experimental Chemistry (Vol. 26)" edited by The Chemical Society of Japan, Maruzen, 1992. In addition, reduction of halogen(s) on the aryl and/or heteroaryl ring may be carried out using the reaction conditions described in Synthesis 1982, 10, 876-878.

### (3) Amidation and Esterification

Among the compound of formula (I), the compound having an amide group or an ester group can be produced by reacting a compound having an amino group or a hydroxy group as a starting material with a carboxylic acid or a reactive derivative thereof. The reaction can be carried out referring, for example, to the methods described in "The fourth edition of Courses in Experimental Chemistry (Vol 22)" edited by The Chemical Society of Japan, Maruzen, 1992 and "The fifth edition of Courses in Experimental Chemistry (Vol. 16)" edited by The Chemical Society of Japan, Maruzen, 2005; or "Compendium of Organic Synthetic Methods", Vols. 1 to 3, or the like.

### (Production process of starting compound)

Of the starting compounds for Production Process 1 above, the starting compound (III) can be produced by the coupling of the indole ring to the benzene ring, for example in accordance with a method described in Journal of the American Chemical Society 2001, 123, 7727-7729.

Further, in the production of the starting compounds in Production Processes 2 to 5 above, an amino group of the starting compound can be prepared by reduction of a nitro group. The reaction can be carried out referring to the methods described in "The fourth eddition of Courses in Experimental Chemistry (Vol. 26)" edited by The Chemical Society of Japan, Maruzen, 1992.

### (Pharmacological test)

An excellent selective inhibitory activity against human 17βHSD type 5 of the compounds of the present invention was confirmed by the test method described below. In this connection, the test may be carried out by referring to the details of test procedure described in Maniatis, T. et al., Molecular Cloning-A Laboratory Manual Cold Spring Harbor Laboratory, NY (1982) and the like. In addition, genes encoding human 17βHSD type5 and type3 described in Sections I and 2 below, and human 17βHSD type 5 and type 3 may be obtained by the method described in Molecular Endocrinology, 1997, 11(13), 1971-1984.

### 1. Isolation of gene encoding human 17βHSD type 5 and enzyme purification

A full-length cDNA encoding human 17βHSD type 5 used in the pharmacological test of the present invention was obtained by the PCR method using a cDNA derived from a human lung cancer-derived cell line, A549 cells as a template. The nucleotide sequence of the obtained cDNA was analyzed by the dideoxyterminator method, and the clone matched with the known human 17βHSD type 5 sequence (GenBank accession No. NM_003739) was selected. *Escherichia coli* BL21 was transformed with a plasmid containing the cDNA and cultured on a large scale. The proteins were purified by using GSTrapFF column (manufactured by Amersham) and PreScissionProtease (manufactured by Amersham). The purification method was carried out in accordance with the instructions attached to the GSTrapFF column.

### 2. Isolation of gene encoding human 17βHSD type 3 and enzyme purification

A full-length cDNA encoding human 17βHSD type 3 used in the pharmacological test of the present invention was obtained by the PCR method using a cDNA derived from human testis as a template. The nucleotide sequence of the obtained cDNA was analyzed by the dideoxyterminator method, and the clone matched with the known human 17βHSD type 3 sequence (GenBank accession No. BC034281) was selected. Subsequently, human fetus kidney-derived cell line, 293 cells, were transformed with a plasmid containing the cDNA, and the cells were collected 24 hours later. The collected cells were then disrupted in a phosphate buffer solution containing 5% glycerol (500 µL per 100 mm-dish of a phosphate buffer solution (pH 7.4, 200 mM) containing 5% glycerol) and centrifuged (16000 rpm, 5 min, 4°C), and the supernatant was used as an enzyme source.

### 3. Measurement of enzyme activities of human 17βHSD type 5 and type 3

Enzyme activity was measured referring to Trevor M. Penning, et al., Biochem. J., 351, 67-77, (2000). Specifically, using a 100 mM potassium phosphate buffer (pH 6.0), (1) the enzyme purified in Section 1 at a final concentration of 10 µg/mL, (2) androstenedione at a final concentration of 300 nM, (3) NADPH at a final concentration of 200 µM, and (4) a test substance, were mixed to react at room temperature for 2 hours, and then the amount of testosterone produced was measured using DELFIA (registered trademark) Testosterone Reagents R050-201 (manufactured by PerkinElmer). The measurement was performed in accordance with the attached instructions. The amount of reduction of testosterone production in the presence of the compound was obtained as a relative value with respect to the amount of testosterone in the absence of the enzyme set at 0% and the amount of testosterone produced in the absence of the compound set at 100%. Then, IC₅₀ values were calculated by the Logistic regression method.

Subsequently, LNCaP cells expressing human 17βHSD type 5 were constructed from a human prostate cancer-derived cell line, LNCaP cells, and a cell growth inhibitory activity of the compound of the present invention was evaluated.

### 4. Construction of UNCaP cells expressing human 17βHSD type 5

A full-length cDNA encoding human 17βHSD type 5 used in the pharmacological test of the present invention was obtained by the PCR method using a cDNA derived from a human lung cancer-derived cell line, A549 cells, as a template. The nucleotide sequence of the obtained cDNA was analyzed by the dideoxyterminator method, and the clone matched with the known human 17βHSD type 5 sequence (GenBank accession No. NM_003739) was selected. The human prostate cancer-derived cell line LNCaP cells were transformed with a plasmid containing the cDNA and the cell line showing stable expression was obtained.

### 5. Measurement of cell growth capability using LNCaP cells expressing human 17βHSD type 5

9000 cells/well of the transformed cells obtained in Section 4 above were seeded in a 96-well plate and cultured overnight. Then, androstenedione, in conjunction with a test compound, was added thereto at a final concentration of 10 nM, followed by culturing for 7 days. After the culturing, number of the cells was counted using a CellTiter-Glo (registered trademark) Luminescent Cell Viability Assay (Promega). The CellTiter-Glo (registered trademark) Luminescent Cell Viability Assay is a reagent that measures the number of the cells by monitoring an intracellular ATP level from the luminescence intensity by luciferase. The experimental manipulation was carried out in accordance with the attached instructions. The cell growth inhitory activity in the presence of a test compound was calculated as a relative value with respect to the number of the cells in the absence of androstenedione set at proliferation of 0%, and the number of the cells in the presence of androstenedione and in the absence of the test compound set at proliferation of 100%. Then, IC₅₀ values were calculated by the Logistic regression method.

Table 1 shows the IC₅₀ values of inhibitory activity against human 17βHSD type 5 and type 3 of the Example compounds included in the compounds of the present invention, and the IC₅₀ values of cell growth inhibitory activity using human 17βHSD type 5-expressing LNCaP cells. Abbreviation "Ex" represents Example number.

**[Table 1]**

| Ex | Type5 IC₅₀ (nM) | Type3 IC₅₀ (nM) | LNCaP-17β5 IC₅₀ (nM) |
|---|---|---|---|
| 1 | 200 | >10,000 | 6,677 |
| 6 | 50 | 1,800 | 46 |
| 17 | 180 | 6,400 | 66 |
| 20 | 120 | 7,300 | 101 |
| 21 | 110 | 4,700 | 102 |
| 32 | 120 | >10,000 | 46 |
| 38 | 96 | >10,000 | 45 |
| 44 | 40 | >10,000 | 34 |
| 47 | 180 | 2,500 | 92 |
| 54 | 70 | 3,200 | 141 |

As shown by the test results above, the compounds of formula (I) hardly have an inhibitory activity against human 17βHSD type 3 and have an inhibitory activity selective to human 17βHSD type 5.

Further, as shown by the test results above, since the compounds of formula (I) have very weak inhibitory activity against human 17βHSD type 3, they are expected to suppress intracrine testosterone synthesis selectively in the prostate by their selective inhibitory effects against 17βHSD type 5 without affecting biosynthesis of testosterone derived from human 17βHSD type 3 in the testes, thus useful for treating and/or preventing benign prostatic hyperplasia and prostate cancer without adverse effects.

Further, as shown by the test results above, since the compounds of formula (I) exhibit a cell growth inhitory activity in the human 17βHSD type 5-expressing LNCaP cells, they suppress intracrine testosterone synthesis selectively in prostate cancer by their selective inhibitory effects against 17βHSD type 5, thus can be used for treating and/or preventing prostate cancer without adverse effects.

Further, a commercial package is also useful which contains the above-mentioned pharmaceutical composition and a description including the above-mentioned effects.

A preparation containing one or two or more kinds of the compound of formula (I) or a salt thereof as an active ingredient can be prepared in accordance with a generally used method, using a pharmaceutical carrier, excipient, or the like, that is usually used in the art.

The administration can be carried out in any form of oral administration via tablets, pills, capsules, granules, powders, liquid preparations, or the like, or parenteral administration via injections such as intraarticular injection, intravenous injection, intramuscular injection, or the like, as well as suppositories, eye drops, eye ointments, percutaneous liquid preparations, ointments, percutaneous patches, transmucosal liquid preparations, transmucosal patches, inhalations, and the like.

As solid compositions for oral administration according to the present invention, tablets, powders, granules, or the like are used. In such a solid composition, one or two or more kinds of active ingredients are mixed with at least one inert excipient such as lactose, mannitol, glucose, hydroxypropylcellulose, microcrystalline cellulose, starch, polyvinyl pyrrolidone, and/or magnesium aluminometasilicate. According to a conventional method, the composition may contain inert additives such as a lubricant such as magnesium stearate, a disintegrator such as sodium carboxymethyl starch, a stabilizing agent, and a solubilizing agent. As occasion demands, the tablets or the pills may be coated with a sugar coating, or a film of gastric or enteric materials.

Liquid compositions for oral administration include pharmaceutically acceptable emulsions, solutions, suspensions, syrups, elixirs, or the like, and contain a generally used inert diluent such as purified water or ethanol. In addition to the inert diluent, the liquid composition may contain an adjuvant such as a solubilizing agent, a moistening agent, and a suspending agent, a sweetener, a flavor, an aroma, and an antiseptic.

Injections for parenteral administration include sterile aqueous or non-aqueous solutions, suspensions and emulsions. The aqueous solvent includes, for example, distilled water for injection and physiological saline. Examples of the non-aqueous solvent include propylene glycol, polyethylene glycol, vegetable oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent. These are sterilized, for example, by filtration through a bacteria-retaining filter, blending of a sterilizing agent, or irradiation. In addition, these can also be used by preparing a sterile solid composition, and dissolving or suspending it in sterile water or a sterile solvent for injection prior to use.

External preparations include ointments, plasters, creams,jellies, cataplasms, sprays, lotions, eye drops, eye ointments, and the like. Generally used ointment bases, lotion bases, aqueous or non-aqueous liquids, suspensions, emulsions, and the like are included. Examples of the ointment or lotion bases include polyethylene glycol, propylene glycol, white Vaseline, bleached beewax, polyoxyethylene hydrogenated castor oil, glyceryl monostearate, stearyl alcohol, cetyl alcohol, lauromacrogol, sorbitan sesquioleate, and the like.

As the transmucosal preparations such as inhalations and transnasal preparations, a solid, liquid or semi-solid form are used, and can be prepared in accordance with a conventionally known method. For example, a known excipient, and also a pH-adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device. The dry powder inhalation devices or the like may be for single or multiple administration use, and a dry powder or a powder-containing capsule can be used. Alternatively, it may be in a form such as a pressurized aerosol spray or the like which uses an appropriate propellant, for example, a suitable gas such as chlorofluoroalkane, hydrofluoroalkane, or carbon dioxide and the like.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compounds of formula (I) can be used in combination with various agents for treating and/or preventing the diseases for which the compounds of formula (I) are considered to be effective. The combined preparation may be administered simultaneously, or separately and continuously or at a desired time interval. The preparations to be co-administered may be a blend, or may be prepared individually.

### (Examples)

The production processes for compounds of formula (I) as an active ingredient of the present invention will be described below as Examples. Production processes for novel compounds among the compounds used as starting materials of compounds of formula (I) will be described as Production Examples. The production processes for the compound of formula (I) are not limited to the production processes in specific Examples shown below and can be produced by a combination of these production precesses or known production processes.

The following abbreviations are used in Production Examples, Examples and Tables below.
Ex: Example number, REx: Production Example number, Data: physicochemical data (FAB+: FAB-MS (M+H)⁺, PAB-: FAB-MS (M-H)⁻, ESI+: ESI-MS (M+H)⁺, ESI-: ESI-MS (M-H)-, API+: API-ES-MS (M+H)⁺, EI: EI-MS (M)⁺, NMR-DMSOd6: δ(ppm) of peak(s) in ¹H NMR in DMSO-d₆), Str: Structural formula, Syn (REx): Production Example numbers in which the corresponding compounds were produced using the same method, Syn (Ex): Example numbers in which the corresponding compounds were produced using the same method, DME: dimethoxyethane, DMF: N,N-dimethylformamide, DMSO: dimethyl sulfoxide, THF: tetrahydrofuran, MeCN: acetonitrile, MeOH: methanol, tBuOH: tert-butyl alcohol, n-BuLi: n-butyllithium, RT: retention time (minutes) in HPLC.

### Production Example I

A mixture of 5.03 g of 2-fluoro-4-methoxy-1-nitrobenzene and 100 mL of THF was cooled to -50°C, and 90 mL of 1M vinyl magnesium bromide-THF solution was added thereto at -30°C or lower, followed by stirring at -50°C for 2 hours. To the reaction solution were added 100 mL of a saturated aqueous ammonium chloride solution and 90 mL of 1M hydrochloric acid, followed by warming to room temperature and stirring for 15 minutes. The mixture was extracted twice with 100 mL of ethyl acetate. The extract was washed with water and saturated brine and dried over anhydrous sodium sulfate, and then the solvent was evaporated under reduced pressure. The residue was purified by silica gel column chromatography (ethyl acetate-hexane=1:20→1:9) to obtain 470 mg of 7-fluoro-5-methoxy-1H-indole as a brown oily susbstance.

### Production Example 2

To a mixture of 1.35 g of ethyl 3-phenyl-5-(trifluoromethyt)-1H-indole-2-carboxylate, 10 mL of methanol and 10 mL of THF was added 6.0 mL of a 1M aqueous sodium hydroxide solution, followed by stirring at 50°C for 3 hours. 30 mL of water and 6 mL of 1M hydrochloric acid were added to the reaction solution, and the precipitated solid was collected by filtration and dried. The solid obtained was dissolved in 15 mL of N-methyl-2-pyrrolidinone, and 100 mg of copper powder was added thereto, followed by stirring at 160°C overnight. The reaction solution was allowed to cool, and 100 mL of ethyl acetate was added thereto to remove insoluble materials, followed by washing with water and saturated brine and drying over anhydrous sodium sulfate, and then the solvent was evaporated under reduced pressure. The residue was purified by silica gel column chromatography (ethyl acetate-hexane=1:4) to obtain 770 mg of3-phenyl-5-(trifluoromethyl)-1H-indole as a brown oily susbstance.

### Production Example 3

A mixture of 120 mg of copper (I) iodide, 160 mg of trans-N,N'-dimethylcyclohexane-1,2-diamine and 6 mL of dioxane were added with 1.2 mL of ethyl 3-iodobenzoate, 850 mg of 111-indole-5-carbonitrile and 1.65 g of potassium carbonate, followed by stirring at 110°C overnight. The reaction solution was allowed to cool, 50 mL of ethyl acetate was added thereto to remove insoluble materials, and the solvent was evaporated under reduced pressure. The residue was purified by silica gel column chromatography (eluent: chloroform), and washed with ether-hexane (1:5) to obtain 1.41 g of ethyl 3-(5-cyano-1H-indol-1-yl) benzoate as a white solid.
Compounds of Production Examples 4 to 10 listed in Tables 2 and 3 were obtained in the same manner as in Production Example 3.

### Production Example 11

A mixture of 1.0 g of 4-fluoro-3-nitrobenzonitrile, 1.09 g of ethyl 3-aminobenzoate, 1.25 g of potassium carbonate and 10 mL of DMF was stirred at 100°C for 3 hours. 60 mL of water was added to the reaction solution. The mixture was extracted with 120 mL of ethyl acetate. The extract was washed with water and saturated brine and dried over anhydrous sodium sulfate, and then the solvent was evaporated under reduced pressure. The residue was washed with ethanol to obtain 1.43 g of ethyl 3-[(4-cyano-2-nitrophenyl)amino] benzoate as a brown solid.
A Compound of Production Example 12 listed in Table 3 was obtained in the same manner as in Production Example 11.

### Production Example 13

To a mixture of 85 mg of palladium acetate, 350 mg of 2,2'-bis(diphenylphosphino)-1,1'-binaphthyl and 15 mL of toluene were added 1.8 mL of ethyl 3-bromobenzoate, 1.02 g of 2-nitroaniline and 4.81 g of cesium carbonate, followed by stirring at 110°C for 5 hours. The reaction solution was allowed to cool, 50 mL of ethyl acetate was added thereto to remove insoluble materials, and the solvent was evaporated under reduced pressure. The residue was purified by silica gel column chromatography (eluent: ethyl acetate-hexane=1:9) to obtain 2.10 g of ethyl 3-[(2-nitrophenyl)amino] benzoate as an light orange oily susbstance.
Compounds of Production Examples 14 to 16 listed in Table 3 were obtained in the same manner as in Production Example 13.

### Production Example 17

To a mixture of 1.42 g of ethyl 3-[(4-cyano-2-nitrrophenyl)amino] benzoate and 10 mL of acetic acid was added 0.77 g of iron powder, followed by stirring at 80°C for 2 hours. Insoluble materials were removed from the reaction solution, and the solvent was evaporated under reduced pressure. 100 mL of a saturated aqueous sodium hydrogen carbonate solution was added to the residue, followed by extraction with 50 mL of chloroform twice. The extract was dried over anhydrous sodium sulfate, and then the solvent was evaporated under reduced pressure to obtain 1.35 g of ethyl 3-[(2-amino-4-cyanophenyl)amino] benzoate as a pale yellow solid.
Compounds of Production Examples 18 to 20, 76 and 77 listed in Tables 4 and 13 were obtained in the same manner as in Production Example 17.

### Production Example 21

fo a mixture of 400 mg of ethyl 3-[(2-amino-4-cyanophenyl)amino] benzoate and 5 mL of THF was added 0.12 mL of acetyl chloride, followed by stirring at room temperature for 2 hours and at 70°C for 2 hours. The reaction solution was allowed to cool, and 30 mL of a saturated aqueous sodium hydrogen carbonate solution was added thereto, followed by extraction with 60 mL of ethyl acetate. The extract was washed with water and saturated brine and dried over anhydrous sodium sulfate, and then the solvent was evaporated under reduced pressure. The residue was purified by silica gel column chromatography (eluent: ethyl acetate-hexane=1:1) to obtain 360 mg of ethyl 3-(5-cyano-2-methyl 1H-benzimidazol-1-yl) benzoate as a white solid.
Compounds of Production Examples 22 to 48 listed in Tables 4 to 8 were obtained in the same manner as in Production Example 21.

### Production Example 49

To a solution of 315 mg of ethyl 3-[(2-amino-4-cyanophenyl)amino] benzoate in 6.3 mL of THF was added 232 mg of (3-fluorophenyl) acetyl chloride, followed by stirring at room temperature for 2.5 hours, and at 80°C for 16 hours. The reaction solution was allowed to cool, and 20 mL of a saturated aqueous sodium hydrogen carbonate solution was added thereto, followed by extraction with 50 mL of ethyl acetate. The extract was washed with water and saturated brine and dried over anhydrous magnesium sulfate, and then solvent was evaporated under reduced pressure. The resulting residue was purified by silica gel column chromatography (eluent: ethyl acetate-hexane=1:6) to obtain 189 mg of ethyl 3-[5-cyano-2-(3-fluorobenzyl)-1H-benzimidazol-1-yl] benzoate as a colorless oily susbstance.
Compounds of Production Examples 50 to 55 listed in Tables 9 and 10 were obtained in the same manner as in Production Example 49.

### Production Example 56

To a mixture of 320 mg of ethyl 3-{[2-amino-4-(trifluoromethyl)phenyl]amino} benzoate and 4 mL of ethanol was added 310 mg of formamidine acetate, followed by stirring under heating to reflux overnight. The reaction solution was allowed to cool, and 30 mL of a saturated aqueous sodium hydrogen carbonate solution was added thereto, followed by extraction with 60 mL of ethyl acetate. The extract was washed with water and saturated brine and dried over anhydrous sodium sulfate, and then the solvent was evaporated under reduced pressure. The residue was purified by silica gel column chromatography (eluent: chloroform) and washed with hexane to obtain 290 mg of ethyl 3-[5-(trifluoromethyl)-1H-benzimidazol-1-yl] benzoate as a pale yellow solid.
A compound of Production Example 57 listed in Table 10 was obtained in the same manner as in Production Example 56.

### Production Example 58

To a solution of 545 mg of ethyl 3-{[2-amino-4-(trifluoromethyl)phenyl]amino} benzoate in 10 mL of acetonitrile were added 0.468 mL of triethylamine and 409 mg of 1,1-carbonylbis (1H-imidazole), followed by stirring at 90°C for 7 hours. After being cooled room temperature, the resulting precipitate was collected by filtration to obtain 499 mg of ethyl 3-[2-oxo-5-(trifluoromethyl)-2,3-dihydro-1H-benzimidazol-1-yl] benzoate as a white solid.

### Production Example 59

To a solution of 889 mg of ethyl 3-[2-oxo-5-(trifluoromethyl)-2,3-dihydro-1H-benzimidazol-1-yl] benzoate in 10 mL of phosphorus oxychloride was added 793 mg of phosphorus pentachloride, followed by stirring at 100°C for 15 hours. After being cooled to room temperature, the reaction solution was concentrated under reduced pressure. Ethyl acetate and saturated aqueous sodium bicarbonate were added to the resulting residue, and the organic layer was separated and washed with saturated brine. The organic layer was dried over anhydrous sodium sulfate, and then the solvent was evaporated under reduced pressure to obtain 985 mg of ethyl 3-[2-chloro-5-(trifluoromethyl)-1H-benzimidazol-1-yl] benzoate as a pale yellow oily susbstance.

### Production Example 60

To a solution of 588 mg of ethyl 3-[2-chloro-5-(trifluoromethyl)-1H-benzimidazol-1-yl] benzoate in 5 mL of methanol was added 0.920 mL of 28% sodium methoxide methanol solution, followed by stirring at room temperature for 16 hours. The resulting precipitate in the reaction solution was collected by filtration to obtain 384 mg of methyl 3-[2-methoxy-5-(trifluoromethyl)-1H-benzimidazol-1-yl] benzoate as a white solid.

### Production Example 61

To a solution of 111 mg of ethyl 3-[2-chloro-5-(trifluoromethyl)-1H-benzimidazol-1-yl] benzoate in 3 mL of THF was added 0.330 mL of a 1M aqueous sodium hydroxide solution, followed by stirring at room temperature for 15 hours. 0.330 mL of a 1M aqueous hydrochloric acid solution was added under ice cooling to the reaction solution, and ethyl acetate and saturated brine were added thereto, followed by separation of the organic layer. The organic layer was dried over anhydrous sodium sulfate, and then the solvent was evaporated under reduced pressure to obtain 113 mg of 3-[2-chloro-s-(trifluoromethyl)-1H-benzimidazol-1-yl] benzoic acid as a white solid.

### Production Example 62

To a mixture of 1.15 g of ethyl 3-[2-pyridin-4-yl-s-(trifluoromethyl)-1H-benzimidazol-1-yl] benzoate, 0.5 mL of concentrated hydrochloric acid and 15 mL of ethanol was added 0.15 g of platinum (IV) oxide, followed by stirring overnight at under a hydrogen atmosphere and at room temperature. The reaction mixture was filtered and then the filtrate was concentrated under reduced pressure. 60 mL of ethyl acetate and 30 mL of a saturated aqueous sodium hydrogen carbonate solution were added to the residue, and the organic layer was separated. The organic layer was washed with water and saturated brine and dried over anhydrous sodium sulfate, and then the solvent was evaporated under reduced pressure. The residue was purified by silica gel column chromatography (eluent: chloroform-methanol-concentrated aqueous ammonia=20:1:0.1→10:1:0.1) to obtain 1.05 g of ethyl 3-[2-piperidin-4-yl-5-(trifluoromethyl)-1H-benzimidazol-1-yl] benzoate as a white amorphous solid.

### Production Example 63

To a mixture of 420 mg of ethyl 3-[2-piperidin-4-yl-5-(trifluoromethyl)-1H-benzimidazol-1-yl] benzoate, 0.17 mL of triethylamine and 4 mL of THF was added 0.12 mL of acetic anhydride, followed by stirring at room temperature for 5 hours. 60 mL of ethyl acetate was added to the reaction solution. The mixture was washed with water and saturated brine and dried over anhydrous sodium sulfate, and the solvent was evaporated under reduced pressure. The residue was purified by silica gel column chromatography (eluent: chloroform-methanol=100:1) to obtain 430 mg of ethyl 3-[2-(1-acetylpiperidin-4-yl)-5-(trifluoromethyl)-1H-benzimidazol-1-yl] benzoate as a white amorphous solid.

### Production Example 64

272 mg of ethyl 3-[2-(4-bromobenzyl)-5-(trifluoromethyl)-1H-benzimidazol-1-yl] benzoate was dissolved in 2.7 mL, of ethanol, and 13.6 mg of palladium-carbon was added thereto, followed by stirring under a hydrogen atmosphere, and at room temperature for 3 hours. The reaction mixture was filtered, and the filtrate was concentrated under reduced pressure. The resulting residue was dissolved in ethyl acetate, and washed with aqueous sodium bicarbonate, water and saturated brine. This was dried over anhydrous magnesium sulfate, and the solvent was evaporated under reduced pressure. The resulting residue was purified by silica gel column chromatography (eluent: ethyl acetate-hexane=1:6) to obtain 229 mg of ethyl 3-[2-benzyl-5-(trifluoromethyl)-1H-benzimidazol-1-yl] benzoate as a pale violet oily susbstance.

### Production Example 65

To a mixture of 300 mg of ethyl 3-{[2-amino-4-(trifluoromethyl)phenyl]amino} benzoate, 0.11 mL of pyridine and 3 mL of THF was added 0.16 mL, of cyclohexanecarbonyl chloride, followed by stirring at room temperature for 1 hour, and at 80°C for 5 hours. The reaction solution was allowed to cool, 30 mL of water was added thereto, and the precipitated solid was collected by filtration. The solid obtained was washed with hexane to obtain 360 mg of ethyl 3-({2-[(cyclohexylcarbonyl)amino]-4-(trifluorotnethyl)phenyl}amino) benzoate as a pale yellow solid.
Compounds of Production Examples 66 to 70 listed in Tables 11 and 12 were obtained in the same manner as in Production Example 65.

### Production Example 71

To a mixture of 340 mg of ethyl 3-({2-[(cyclohexylcarbonyl)amino]-4-(trifluoromethyl)phenyl}amino) benzoate, 2.5 mL of methanol and 2.5 mL of THF was added 1.1 mL of a 1M aqueous sodium hydroxide solution, followed by stirring at 50°C for 3 hours. 1.1 mL of 1M hydrochloric acid and 30 mL of water were added to the reaction solution, and the precipitated solid was collected by filtration. The solid obtained was washed with ethanol to obtain 210 mg of 3-({2-[(cyclohexylcarbonyl)amino]-4-(trifluoromethyl)phenyl}amino) benzoic acid as a white solid.
Compounds of Production Examples 72 to 74, 78 and 79 listed in Tables 12 and 13 were obtained in the same manner as in Production Example 71.

### Production Example 75

To a mixture of 324 mg of ethyl 3-{[2-amino-4-(trifluoromethyl)phenyl]amino} benzoate in 5 mL of ethanol and THF was added 1.50 mL of a 1M aqueous sodium hydroxide solution, followed by stirring at room temperature for 2 hours. 1.00 mL of 1 M aqueous sodium hydroxide solution was added to the reaction solution, followed by stirring at room temperature for 27 hours. The reaction solution was concentrated under reduced pressure, and 2.50 mL of 1M aqueous hydrochloric acid solution and 10 mL of water were added thereto under ice cooling to the resulting residue. The resulting precipitate was collected by filtration to obtain 281 mg of 3-{[2-amino-4-(trifluoromethyl)phenyl]amino} benzoic acid as a brown solid.

**[Table 2]**

| REx | Syn (Rex) | Str | Data |
|---|---|---|---|
| 1 | 1 | 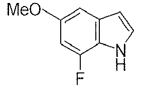 | EI: 165 |
| 2 | 2 | 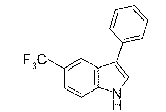 | EI: 261 |
| 3 | 3 | 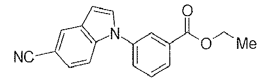 | FAB+: 291 |
| 4 | 3 | 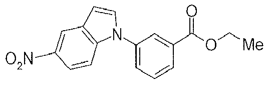 | FAB+: 311 |
| 5 | 3 | 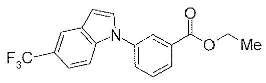 | FAB+: 334 |
| 6 | 3 | 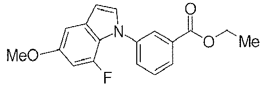 | EI: 313 |
| 7 | 3 | 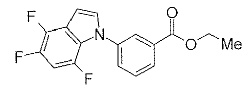 | EI: 319 |
| 8 | 3 | 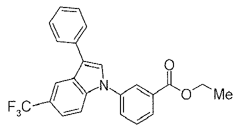 | FAB+: 410 |

**[Table 3]**

| REx | Syn (REx) | Str | Data |
|---|---|---|---|
| 9 | 3 | 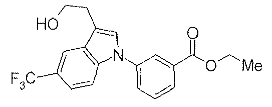 | FAB+: 378 |
| 10 | 3 | 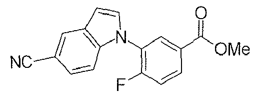 | CI+: 295 |
| 11 | 11 | 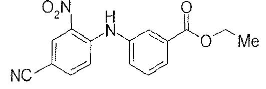 | ESI-: 310 |
| 12 | 11 | 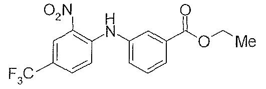 | FAB-: 353 |
| 13 | 13 | 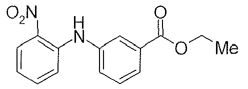 | Fay-: 286 |
| 14 | 13 | 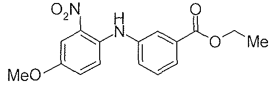 | FAB-: 315 |
| 15 | 13 | 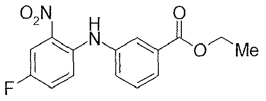 | Fay-: 304 |
| 16 | 13 | 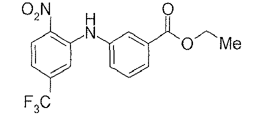 | FAB+: 354 |

**[Table 4]**

| REx | Syn (REx) | Str | Data |
|---|---|---|---|
| 17 | 17 | 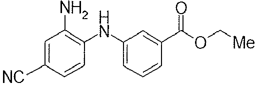 | FAB+: 282 |
| 18 | 17 | 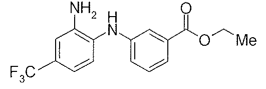 | FAB-: 323 |
| 19 | 17 | 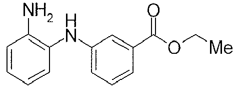 | EI: 256 |
| 20 | 17 | 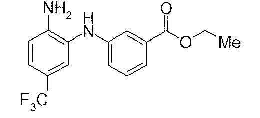 | EI: 324 |
| 21 | 21 | 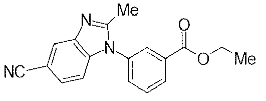 | FAB+: 306 |
| 22 | 21 | 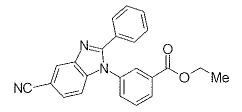 | ESI+: 368 |
| 23 | 21 | 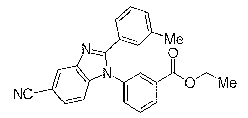 | FAB+: 382 |

**[Table 5]**

| REx | Syn (REx) | Str | Data |
|---|---|---|---|
| 24 | 21 | 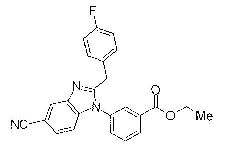 | ESI+: 400 |
| 25 | 21 | 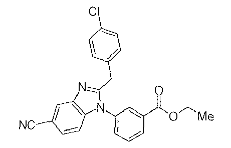 | ESI+: 416 |
| 26 | 21 | 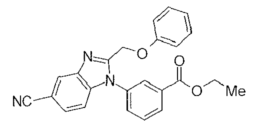 | ESI+: 398 |
| 27 | 21 | 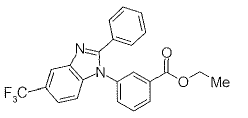 | ESI+: 411 |
| 28 | 21 | 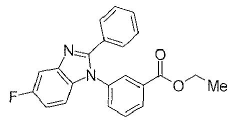 | FAB+: 361 |
| 29 | 21 | 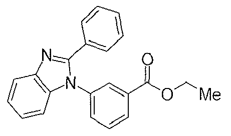 | EI: 342 |

**[Table 6]**

| REx | Syn (REx) | Str | Data |
|---|---|---|---|
| 30 | 21 | 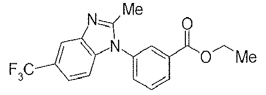 | ESI+: 349 |
| 31 | 21 | 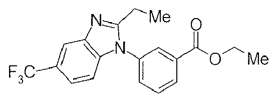 | FAB+: 363 |
| 32 | 21 | 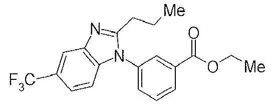 | FAB+: 377 |
| 33 | 21 | 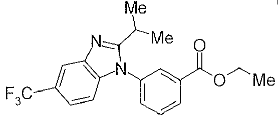 | FAB+: 377 |
| 34 | 21 | 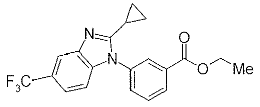 | FAB+: 375 |
| 35 | 21 | 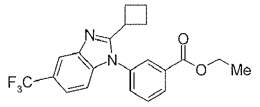 | FAB+: 389 |
| 36 | 21 | 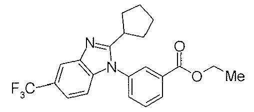 | FAB+: 403 |

**[Table 7]**

| REx | Syn (REx) | Str | Data |
|---|---|---|---|
| 37 | 21 | 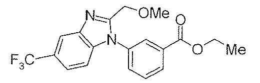 | FAB+: 379 |
| 38 | 21 | 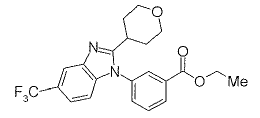 | FAB+: 419 |
| 39 | 21 | 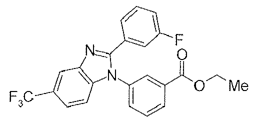 | FAB+: 429 |
| 40 | 21 | 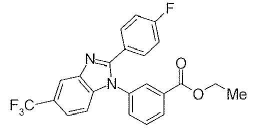 | FAB+: 429 |
| 41 | 21 | 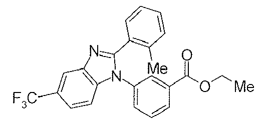 | FAB+: 425 |
| 42 | 21 | 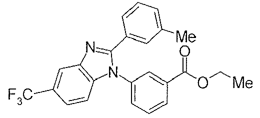 | FAB+: 425 |

**[Table 8]**

| Rex | Syn (REx) | Str | Data |
|---|---|---|---|
| 43 | 21 | 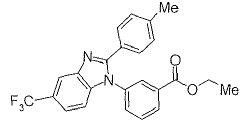 | FAB+: 425 |
| 44 | 21 | 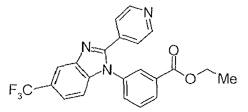 | FAB+: 412 |
| 45 | 21 | 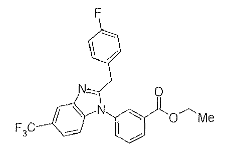 | ESI+ 443 |
| 46 | 21 | 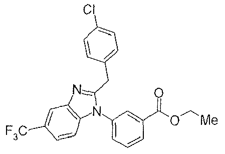 | ESI+: 459 |
| 47 | 21 | 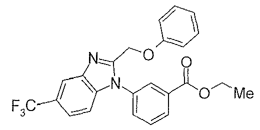 | FAB+: 441 |
| 48 | 21 | 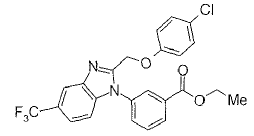 | FAB+: 475 |

**[Table 9]**

| Rex | Syn (REx) | Str | Data |
|---|---|---|---|
| 49 | 49 | 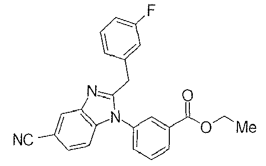 | EST+: 400 |
| 50 | 49 | 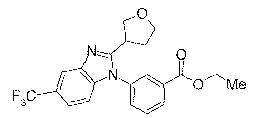 | FAB+: 405 |
| 51 | 49 | 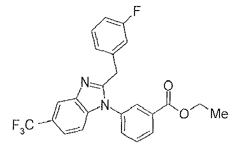 | ESI+: 443 |
| 52 | 49 | 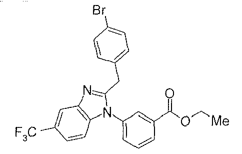 | ESI+: 502 |
| 53 | 49 | 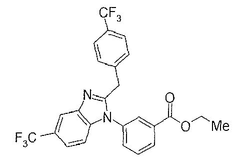 | ESI+: 494 |

**[Table 10]**

| REx | Syn (REx) | Str | Data |
|---|---|---|---|
| 54 | 49 | 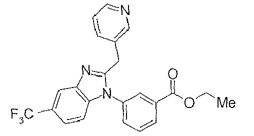 | ESI+: 426 |
| 55 | 49 | 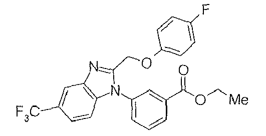 | FAB+: 459 |
| 56 | 56 | 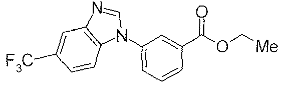 | FAB+: 335 |
| 57 | 56 | 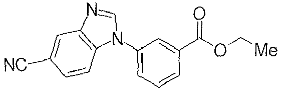 | FAB+: 292 |
| 58 | 58 | 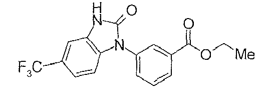 | FAB+: 351 |
| 59 | 59 | 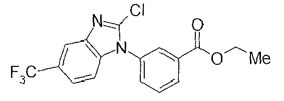 | FAB+: 369 |
| 60 | 60 | 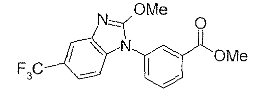 | FAB+: 351 |

**[Table 11]**

| REx | Syn (REx) | Str | Data |
|---|---|---|---|
| 61 | 61 | 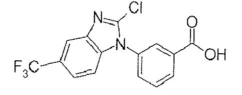 | FAB+: 341 |
| 62 | 62 | 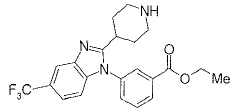 | FAB+: 418 |
| 63 | 63 | 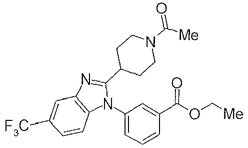 | FAB+: 460 |
| 64 | 64 | 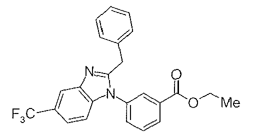 | API+: 425 |
| 65 | 65 | 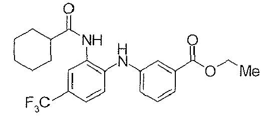 | FAB+: 435 |
| 66 | 65 | 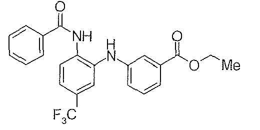 | FAB+: 429 |

**[Table 12]**

| REx | Syn (REx) | Str | Data |
|---|---|---|---|
| 67 | 65 | 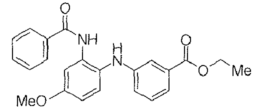 | PAB+: 391 |
| 68 | 65 | 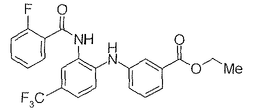 | FAB+: 447 |
| 69 | 65 | 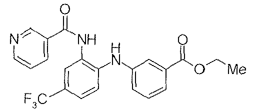 | FAB+: 430 |
| 70 | 65 | 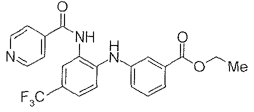 | FAB+: 430 |
| 71 | 71 | 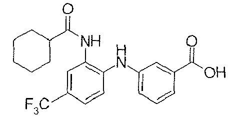 | FAB-: 405 |
| 72 | 71 | 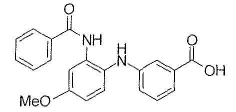 | ESI+: 363 |

**[Table 13]**

| REx | Syn (REx) | Str | Data |
|---|---|---|---|
| 73 | 71 | 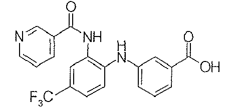 | ESI-: 400 |
| 74 | 71 | 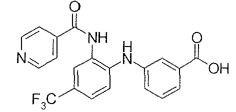 | FAB+: 402 |
| 75 | 75 | 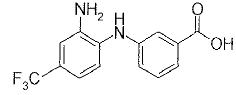 | ESI-: 295 |
| 76 | 17 | 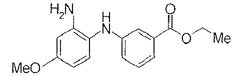 | |
| 77 | 17 | 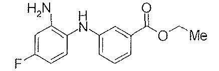 | |
| 78 | 71 | 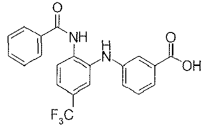 | |
| 79 | 71 | 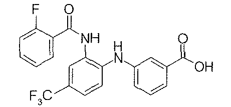 | |

The following Examples are described to explain the present invention in more detail, and the present invention is not restricted to the following Examples. Although the present invention is fully explained by way of the Examples, the person skilled in the art understand that various alterations and modifications can naturally be made.
Accordingly, these alterations and modifications are included in the present invention unless they depart from the scope of the present invention.

### Example I

To a mixture of 1.0 g of ethyl 3-(5-cyano-1H-indol-1-yl) benzoate, 10 mL of methanol and 20 mL of THF was added 5.5 mL of 1 M aqueous sodium hydroxide solution, followed by stirring at room temperature for 3 hours. 5.5 mL of a 1M aqueous hydrochloric acid solution and 50 mL of water were added to the reaction solution, and the precipitated solid was collected by filtration. The solid obtained was washed with ethanol-ether (1:2) to obtain 800 mg of 3-(5-cyano-1H-indol-1-yl) benzoic acid as a white solid.
Compounds of Examples 2 to 46 listed in Tables 14 to 17 were obtained in the same manner as in Example 1.

### Example 47

60 mg of sodium hydride (55%, dispersion in paraffin liquid) was added under ice cooling to a mixture of 150 mg of 3-[3-(2-hydroxyethyl)-5-(trifluoromethyl)-1H-indol-1-yl] benzoic acid, 0.090 mL of methyl iodide and 2 mL of THF, followed by stirring under ice cooling for 3 hours and at room temperature overnight. 20 mL of water and 10 mL of 5% aqueous potassium hydrogen sulfate solution were added to the reaction solution, and the precipitated solid was collected by filtration. The solid obtained was washed with ether-hexane to obtain 110 mg of 3-[3-(2-methoxyethyl)-5-(trifluoromethyl)-1H-indol-1-yl] benzoic acid as a pale yellow solid.

### Example 48

A mixture of 190 mg of 3-({2-[(cyclohexylcarbonyl)amino]-4-(trifluoromethyl)phenyl}amino) benzoic acid and 4 mL of acetic acid was stirred at 80°C overnight. The solvent was evaporated under reduced pressure, and the residue was washed with ethanol to obtain 160 mg of 3-[2-cyclohexyl-5-(trifluoromethyl)-1H-benzimidazol-1-yl] benzoic acid as a white solid.
Compounds of Examples 49 to 53 listed in Tables 17 and 18 were obtained in the same manner as in Example 48.

### Example 54

0.157 mL of a trifluoroacetic anhydride was added under ice cooling to a solution of 275 mg of 3-{[2-amino-4-(trifluoromethyl)phenyl]amino} benzoic acid in 5 mL of THF, followed by stirring at room temperature for 30 minutes. The reaction solution was concentrated under reduced pressure, and 5 mL of acetic acid was added to the resulting residue, followed by stirring at 90°C for 12 hours. After being cooled to room temperature, the reaction solution was concentrated under reduced pressure. The resulting residue was crystallized from a mixture of hexane and ethyl acetate to obtain 176 mg of 3-[2,5-bis(trifluoromethyl)-1H-benzimidazol-1-yl] benzoic acid as a pale yellow solid.

### Example 55

A solution of 163 mg of 3-[2-chloro-5-(trifluoromethyl)-1H-benzimidazol-1-yl] benzoic acid in 2 mL of DMF was added under ice cooling to a suspension of 163 mg of sodium ethoxide in 3 mL of DMF, followed by stirring at room temperature for 1 hour. 163 mg of sodium ethoxide was added to the reaction solution, followed by stirring at room temperature for 2 hours. 4.8 mL of 1 M aqueous hydrochloric acid solution was added under ice cooling to the reaction solution, and ethyl acetate and water were added thereto, followed by separation of the organic layer. The organic layer was washed with saturated brine and dried over anhydrous sodium sulfate, and then the solvent was evaporated under reduced pressure. The resulting residue was purified by silica gel column chromatography (eluent: chloroform-methanol=24:1), and the solid obtained was recrystallized from a mixture of hexane and ethyl acetate to obtain 202 mg of 3-[2-ethoxy-5-(trifluoromethyl)-1H-benzimidazol-1-yl] benzoic acid as a white solid.
A compound of Example 56 listed in Table 18 was obtained in the same manner as in Example 55.

The physicochemical data of compounds of Examples I to 56 are shown in Tables 19 to 21.

**[Table 14]**

| Ex | Syn (Ex) | Str | Ex | Syn (Ex) | Str |
|---|---|---|---|---|---|
| 1 | 1 | 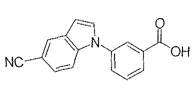 | 2 | 1 | 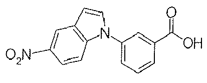 |
| 3 | 1 | 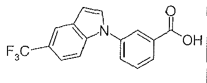 | 4 | 1 | 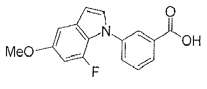 |
| 5 | 1 | 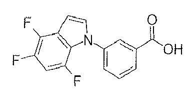 | 6 | 1 | 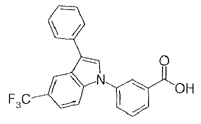 |
| 7 | 1 | 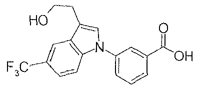 | 8 | 1 | 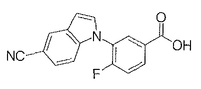 |
| 9 | 1 | 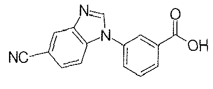 | 10 | 1 | 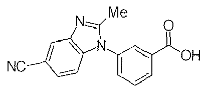 |
| 11 | 1 | 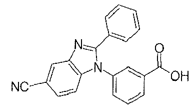 | 12 | 1 | 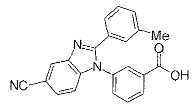 |
| 13 | 1 | 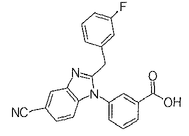 | 14 | 1 | 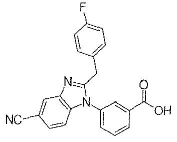 |

**[Table 15]**

| Ex | Syn (Ex) | Str | Ex | Syn (Ex) | Str |
|---|---|---|---|---|---|
| 15 | 1 | 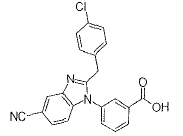 | 16 | 1 | 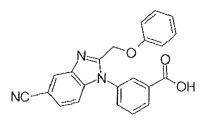 |
| 17 | 1 | 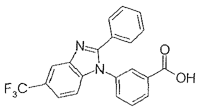 | 18 | 1 | 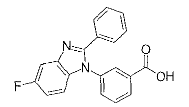 |
| 19 | 1 | 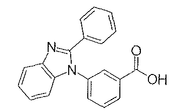 | 20 | 1 | 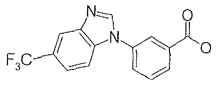 |
| 21 | 1 | 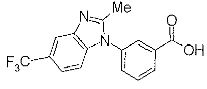 | 22 | 1 | 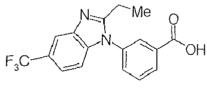 |
| 23 | 1 | 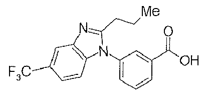 | 24 | 1 | 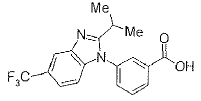 |
| 25 | 1 | 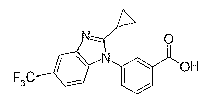 | 26 | 1 | 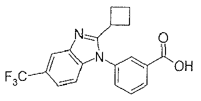 |
| 27 | 1 | 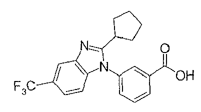 | 28 | 1 | 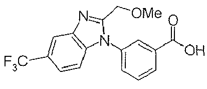 |

**[Table 16]**

| Ex | Syn (Ex) | Str | Ex | Syn (Ex) | Str |
|---|---|---|---|---|---|
| 29 | 1 | 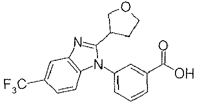 | 30 | 1 | 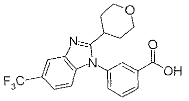 |
| 31 | 1 | 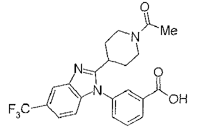 | 32 | 1 | 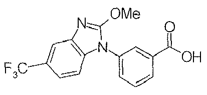 |
| 33 | 1 | 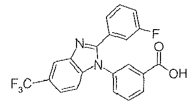 | 34 | 1 | 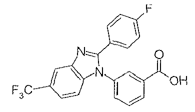 |
| 35 | 1 | 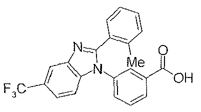 | 36 | 1 | 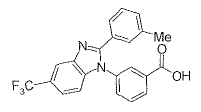 |
| 37 | 1 | 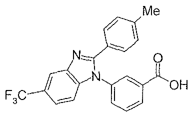 | 38 | 1 | 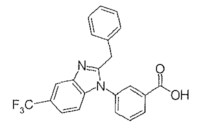 |
| 39 | 1 | 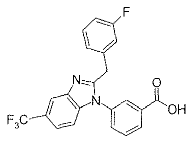 | 40 | 1 | 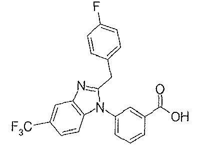 |

**[Table 17]**

| Ex | Syn (Ex) | Str | Ex | Syn (Ex) | Str |
|---|---|---|---|---|---|
| 41 | 1 | 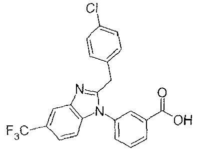 | 42 | 1 | 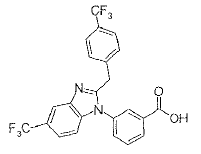 |
| 43 | 1 | 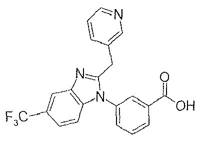 | 44 | 1 | 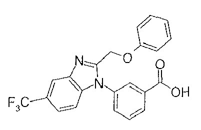 |
| 45 | 1 | 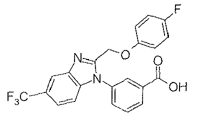 | 46 | 1 | 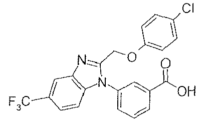 |
| 47 | 47 | 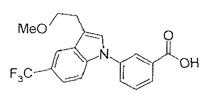 | 48 | 48 | 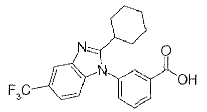 |
| 49 | 48 | 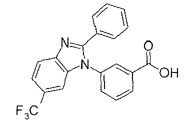 | 50 | 48 | 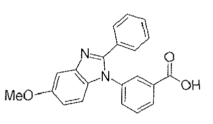 |
| 51 | 48 | 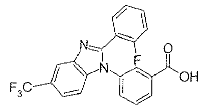 | 52 | 48 | 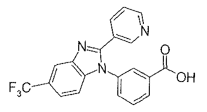 |

**[Table 18]**

| Ex | Syn (Ex) | Str | Ex | Syn (Ex) | Str |
|---|---|---|---|---|---|
| 53 | 48 | 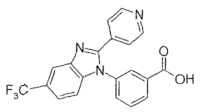 | 54 | 54 | 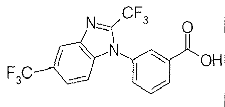 |
| 55 | 55 | 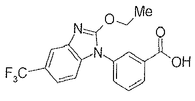 | 56 | 55 | 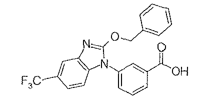 |

**[Table 19]**

| Ex | Data |
|---|---|
| 1 | NMR-DMSOd6: 6.90 (1H, d, J=3.3Hz), 7.58 (1H, dd, J=1.5, 8.6Hz), 7.68 (1H, d, J=8.6Hz), 7.75 (1H, t, J=7.8Hz), 7.88-7.93 (1H, m), 7.96 (1H, d, J=3.3Hz), 8.03 (1H, d, J=7.6Hz), 8.06 (1H, s), 8.24 (1H, d, J=0.8Hz), 13.35 (1H, s) FAB+: 261 |
| 2 | FAB-: 281 |
| 3 | FAB-: 304 |
| 4 | FAB+: 284 |
| 5 | FAB-: 290 |
| 6 | FAB-: 380 |
| 7 | ESI-:348 |
| 8 | FAB+: 281 |
| 9 | ESI+: 264 |
| 10 | ESI+: 278 |
| 11 | ESI+: 340 |
| 12 | FAB+: 354 |
| 13 | ESI+: 372 |
| 14 | ESI+: 372 |
| 15 | ESI+: 388 |
| 16 | ESI+: 370 |
| 17 | NMR-DMSOd6: 7.37-7.49 (4H, m), 7.53-7.57 (2H, m), 7.62 (1H, d, J=8.6Hz), 7.70-7.77 (2H, m), 7.99 (1H, s), 8.10-8.14 (1H, m), 8.20 (1H, s), 13.37 (1H, s) FAB+: 383 |
| 18 | ESI+: 333 |
| 19 | ESI+: 315 |
| 20 | ESI+: 307 |
| 21 | NMR-DMSOd6: 2.48 (3H, s), 7.33 (1H, d, J=8.4Hz), 7.53 (1H, d, J=8.4Hz), 7.81 (1H, t, J=7.8Hz), 7.85-7.90 (1H, m), 8.01 (1H, s), 8.06 (1H, t, J=1.8Hz), 8.16 (1H, d, J=7.8Hz), 13.40 (1H, s) FAB+: 321 |

**[Table 20]**

| Ex | Data |
|---|---|
| 22 | FAB+: 335 |
| 23 | FAB+: 349 |
| 24 | FAB+: 349 |
| 25 | FAB+: 347 |
| 26 | ESI+: 361 |
| 27 | FAB+: 375 |
| 28 | FAB+: 351 |
| 29 | FAB+: 377 |
| 30 | FAB+: 391 |
| 31 | FAB+: 432 |
| 32 | NMR-DMSOd6: 4.17 (3H, s), 7.35 (1H, d, J=8.4Hz), 7.48 (1H, d, J=8.4Hz), 7.76 (1H, t, J=8.0Hz), 7.85-7.87 (2H, m), 8.07-8.10 (2H, m), 13.36 (1H, s) FAB+: 337 |
| 33 | FAB+: 401 |
| 34 | FAB+-: 401 |
| 35 | ISI+: 397 |
| 36 | FAB+: 397 |
| 37 | FAB+: 397 |
| 38 | NMR-DMSOd6: 4.21 (2H, s), 7.04 (2H, d, J= 7.3 Hz), 7.15-7.22 (3H, m), 7.30 (1H, d, J = 8.6 Hz), 7.54 (1H, d, J = 8.5 Hz), 7.73-7.76 (2H, m), 7.89 (1H, m), 8.08 (1H, s), 8.10-8.14 (1H, m), 13.33 (1H, s) ESI+: 397 |
| 39 | ESI+: 415 |
| 40 | ESI+: 415 |
| 41 | ESI+: 431 |
| 42 | ESI-: 463 |
| 43 | ESI+: 398 |
| 44 | NMR-DMSOd6: 5.33 (2H, s), 6.87-6.95 (3H, m), 7.21-7.26 (2H, m), 7.42 (1H, d, J=8.7Hz), 7.63 (1H, dd, J=8.6, 1.5Hz), 7.76 (1H, t, J=7.8Hz), 7.89 (1H, dt, J=8.1, 1.6Hz), 8.12-8.14 (2H, m), 8.18 (1H, s), 13.34 (1H, s) FAB+: 413 |
| 45 | FAB+: 431 |
| 46 | FAB+: 447 |
| 47 | NMR-DMSOd6: 3.06 (2H, t, J=6.8Hz), 3.30 (3H, s), 3.67 (2H, t, J=6.8Hz), 7.52 (1H, d, J=8.8Hz), 7.68-7.78 (3H, m), 7.89 (1H, d, J=7.6Hz), 7.99 (1H, d, J=6.8Hz), 8.05 (1H, s), 8.09 (1H, s), 13.31 (1H, s) FAB-: 362 |

**[Table 21]**

| Ex | Data |
|---|---|
| 48 | NMR-DMSOd6: 1.09-1.31 (3H, m), 1.59-1.77 (5H, m), 1.83-1.91 (2H, m), 2.66-2.75 (1H, m), 7.25 (1H, d, J=8.4Hz), 7.52 (1H d, J=8.4Hz), 7.82 (1H, t, J=7.8Hz), 7.87 (1H, d, J=7.8Hz), 8.03 (1H, s), 8.06 (1H, s), 8.18 (1H, d, J=7.8Hz), 13.31 (1H, s) FAB+:389 |
| 49 | FAB+:383 |
| 50 | FAB+: 345 |
| 51 | FAB+: 401 |
| 52 | FAB+: 384 |
| 53 | FAB+: 384 |
| 54 | ESI+: 375 |
| 55 | ESI+: 351 |
| 56 | ESI+: 413 |

### Example 57

To a solution of 6 mg of isovaleryl chloride in 0.2 mL of THF were added a solution of 13 mg of ethyl 3-[2-amino-4-(trifluorometlyl)phenyl]aminobenzoate and 0.02 mL of pyridine in 0.3 mL of THF, followed by stirring at room temperature overnight. Water and chloroform were added thereto, the organic layer was separated, and the solvent was evaporated under reduced pressure. 0.5 mL of acetic acid was added to the resulting residue, followed by stirring at 90°C overnight and then the solvent was evaporated under reduced pressure. The resulting residue was dissolved in 0.2 mL of methanol, 0.2 mL of THF, 0.1 mL of a 5M aqueous sodium hydroxide solution, followed by stirring at 60°C overnight. After being cooled to room temperature, neutralizing with 1M aqueous hydrochloric acid solution, the solvent was evaporated under reduced pressure. The resulting residue was fractionated and purified by HPLC to obtain 4.1 mg of 3-[2-isobutyl-5-(trifluoromethyl)-1H-benzimidazol-1-yl] benzoic acid.

HPLC conditions used for purification
Column: SunFire (registered trademark) (particle size: 5 µm, inner diameter: 19 mm, and length: 100 mm)
Mobile phase: A sol, methanol, and B sol, 0.1 % aqueous formic acid solution

**[Table 22]**

| Time (min) | A Sol (%) | B Sol (%) |
|---|---|---|
| 0-1 | 10 | 90 |
| 1-9 | 10→95 | 90→5 |
| 9-12 | 95 | 5 |

Flow rate: 25 mL/min
Column temperature: 20°C
Injection volume: 800 µL

The conditions of HPLC performed for the determination of RT are shown below.
Column: Wakosil-II 5C18AR (registered trademark) (particle size: 5 µm, inner diameter: 2.0 mm, and length: 30 mm)
Mobile phase: A sol, 5 mM aqueous trifluoroacetic acid solution, and B sol, methanol

**[Table 23]**

| Time (min) | A Sol (%) | B Sol (%) |
|---|---|---|
| 0-4 | 90→0 | 10→100 |
| 4-4.5 | 0 | 100 |

Flow rate: 1.2 mL/min
Detection wavelength: 254 nm
Column temperature: 35.0°C
Injection volume: 5 µL
Compounds of Examples 58 to 60 listed in Table 24 were obtained in the same manner as in Example 57.

**[Table 24]**

| Ex | Str | RT | ESI+ |
|---|---|---|---|
| 57 | 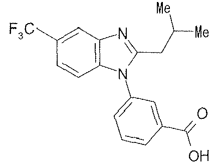 | 2.82 | 363 |
| 58 | 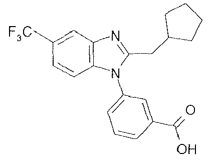 | 3.04 | 389 |
| 59 | 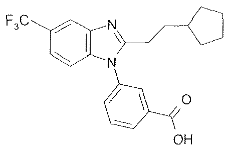 | 3.21 | 403 |
| 60 | 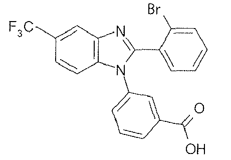 | 2.98 | 461 |

### Industrial Applicability

Since the compound which is an active ingredient of the pharmaceutical of the present invention has a selective inhibitory effect against 17βHSD type 5 and a superior pharmacological effect based thereon, the pharmaceutical composition according to the present invention is useful as an agent for treating and/or preventing diseases associated with 17βHSD type 5, particularly prostate cancer, benign prostatic hyperplasia, acne, seborrhea, hirsutism, baldness, alopecia, precocious puberty, adrenal hypertrophy, polycystic ovary syndrome, breast cancer, lung cancer, endometriosis, leiomyoma, or the like.

## Claims

1. A pharmaceutical composition for preventing or treating a disease associated with 17βHSD type 5, comprising a compound of formula (I) or a salt thereof: 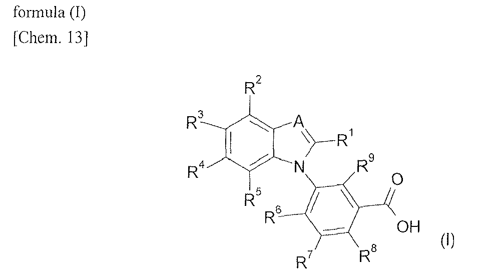 (wherein:
A represents C-R¹⁰ or N;
R¹ represents hydrogen, lower alkyl, halogeno lower alkyl, lower alkyl substituted with lower alkyl-O-, lower alkyl-O-, halogeno lower alkyl-O-, lower alkyl-O- substituted with lower alkyl-O-, cycloalkyl-L- which may be substituted, aryl-L- which may be substituted, or a heterocyclic group-L- which may be substituted;
L represents a bond, lower alkylene, lower alkenylene, O, lower alkylene-O, or O-lower alkylene;
R², R⁴, R⁵, R⁶, R⁷, R⁸, and R⁹, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
R³ represents hydrogen, halogen, cyano, nitro, halogeno lower alkyl, or lower alkyl-O-;
R¹⁰ represents hydrogen, aryl which may be substituted, or lower alkyl which may be substituted with hydroxy or lower alkyl-O-,
provided that,
when A is C-R¹⁰, R¹ represents hydrogen or lower alkyl;
when A is N, and R¹, R², R⁴, R⁵, R⁶, R⁷, R⁸, and R⁹ are hydrogen, R³ represents halogen, cyano, halogeno lower alkyl, or lower alkyl-O-;
when A is N, R¹ is methyl, and R², R³, R⁴, R⁵, R⁶, R⁷, and R⁹ are hydrogen, R⁸ is a group other than [(2E)-3-(2-naphthalenyl)-1-oxo-2-buten-1-yl]amino).

2. A compound of formula (II) or a salt thereof: 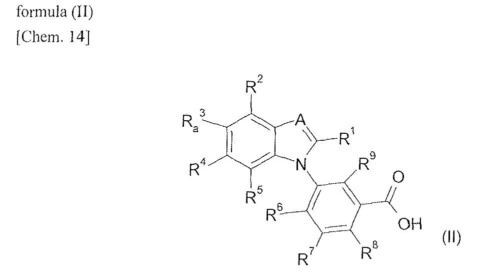 (wherein:
A represents C-R¹⁰ or N;
R¹ represents hydrogen, lower alkyl, halogeno lower alkyl, lower alkyl substituted with lower alkyl-O-, lower alkyl-O-, halogeno lower alkyl-O-, lower allcyl-O- substituted with lower alkyl-O-, cycloalkyl-L- which may be substituted, aryl-L- which may be substituted, or a heterocyclic group-L- which may be substituted;
L represents a bond, lower alkylene, lower alkenylene, O, lower alkylene-O, or O-lower alkylene;
R², R⁴, R⁵, R⁶, R⁷, R⁸, and R⁹, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
Rₐ³ represents hydrogen, halogen, cyano, halogeno lower alkyl, or lower alkyl-O-;
R¹⁰ represents hydrogen, aryl which may be substituted, or lower alkyl which may be substituted with hydroxy or lower alkyl-O-,
provided that,
when A is C-R¹⁰, R¹ represents hydrogen or lower alkyl, but when R¹, R², R⁵, R⁶, R⁷, R⁸, and R⁹ are hydrogen, R⁴ is chloro, and R¹⁰ is isobutyl, Rₐ³ is a group other than hydrogen;
when A is N, and R¹, R², K⁴, R⁵, R⁶, R⁷, R⁸, and R⁹ are hydrogen, Rₐ³ represents halogen, cyano, or lower alkyl-O-;
when A is N, R¹ is methyl, R², R⁴, R⁵, R⁶, R⁷, and R⁹ are hydrogen, and R⁸ is [(2E)-3-(2-naphthalenyl)-1-oxo-2-buten-1-yl]amino, Rₐ³ is a group other than hydrogen;
when A is N, and R¹, R², R⁵, R⁶, R⁷, R⁸, and R⁹ are hydrogen, and R⁴ is tert-butyl, Rₐ³ is a group other than hydrogen;
when A is N, R¹ is hydrogen or methyl, R², R⁶, and R⁷ are hydrogen, R⁴ and R⁸ are hydroxy, and R⁵ is carboxy, Rₐ³ is a group other than hydrogen).

3. The compound according to claim 2, wherein A is C-R¹⁰, or a salt thereof.

4. The compound according to claim 3, wherein R¹ is hydrogen, and R², R⁴, R⁵, R⁶, R⁷, R⁸, and R⁹, which are the same or different from each other, represent hydrogen or halogen, or a salt thereof.

5. The compound according to claim 4, wherein Rₐ³ is halogeno lower alkyl, or cyano, or a salt thereof.

6. The compound according to claim 2, wherein A is N, or a salt thereof.

7. The compound according to claim 6, wherein R², R⁴, R⁵, R⁶, R⁷, R⁸, and R⁹, which are the same or different from each other, represent hydrogen or halogen, or a salt thereof.

8. The compound according to claim 7, wherein Rₐ³ is halogeno lower alkyl, or cyano, or a salt thereof.

9. The compound according to claim 8, wherein Rₐ³ is trifluoromethyl, or a salt thereof.

10. The compound according to claims 6 to 9, wherein R¹ represents hydrogen; lower alkyl; halogeno lower alkyl; lower alkyl substituted with lower alkyl-O-, phenyl or phenyl-O-; phenyl which may be substituted with lower alkyl, halogen, or lower alkyl-O-; or cycloalkyl, wherein the phenyl moiety in the lower alkyl substituted with phenyl or phenyl-O- may be substituted with lower alkyl, halogen, halogeno lower alkyl, or lower alkyl-O-, or a salt thereof.

11. The compound according to claim 9, wherein R¹ is hydrogen, lower alkyl, halogeno lower alkyl, or lower alkyl substituted with lower alkyl-O-, or a salt thereof.

12. The compound according to claim 9, wherein R¹ is lower alkyl-O- which may be substituted with phenyl, or a salt thereof.

13. The compound according to claim 9, wherein R¹ is cycloalkyl, or a salt thereof.

14. The compound according to claim 9, wherein R¹ is phenyl which may be substituted with a group selected from lower alkyl, halogen, and lower alkyl-O-, or a salt thereof.

15. The compound according to claim 9, wherein R¹ is lower alkyl substituted with phenyl or lower alkyl substituted with phenyl-O-, wherein the phenyl may be substituted with a group selected from lower alkyl, halogen, halogeno lower alkyl, and lower alkyl-O-, or a salt thereof.

16. The compound according to claim 9, which is
3-[5-(trifluoromethyl)-1H-benzimidazol-1-yl] benzoic acid (Example 20);
3-[2-methyl-5-(trifluoromethyl)-1H-benzimidazol-1-yl] benzoic acid (Example 21);
3-[2-ethyl-5-(trifluoromethyl)-1H-benzimidazol-1-yl] benzoic acid (Example 22);
3-[2-propyl-5-(trifluoromethyl)-1H-benzimidazol-1-yl] benzoic acid (Example 23);
3-[2,5-bis(trifluoromethyl)-1H-benzimidazol-1-yl] benzoic acid (Example 54);
3-[2-phenoxymethyl-5-(trifluoromethyl)-1H-benzimidazol-1-yl] benzoic acid (Example 44);
3-[2-methoxy-5-(trifluoromethyl)-1H-benzimidazol-1-yl] benzoic acid (Example 32);
3-[2-cyclopropyl-5-(trifluoromethyl)-1H-benzimidazol-1-yl] benzoic acid (Example 25);
3-[2-cyclohexyl-5-(trifluoromethyl)-1H-benzimidazol-1-yl] benzoic acid (Example 48);
3-[2-phenyl-5-(trifluoromethyl)-1H-benzimidazol-1-yl] benzoic acid (Example 17);
3-[2-(3-methylphenyl)-5-(trifluoromethyl)-1H-benzimidazol-1-yl] benzoic acid (Example 36);
3-[2-benzyl-5-(trifluoromethyl)-1H-benzimidazol-1-yl] benzoic acid (Example 38);
3-[2-(3-fluorobenzyl)-5-(trifluoromethyl)-1H-benzimidazol-1-yl] benzoic acid (Example 39);
3-[2-(4-fluorobenzyl)-5-(trifluoromethyl)-1H-benzimidazol-1-yl] benzoic acid (Example 40);
3-[2-(4-chlorobenzyl)-5-(trifluoromethyl)-1H-benzimidazol-1-yl] benzoic acid (Example 41); or
3-[2-(benzyloxy)-5-(trifluoromethyl)-1H-benzimidazol-1-yl] benzoic acid (Example 56),
or a salt thereof.

17. A pharmaceutical composition comprising the compound according to claim 2 or a salt thereof, and a pharmaceutically acceptable excipient.

18. Use of the compound according to claim 1 or a salt thereof, for the manufacture of a pharmaceutical composition for preventing or treating a disease associated with 17βHSD type 5.

19. Use of the compound according to claim 1 or a salt thereof, for the manufacture of a pharmaceutical composition for preventing or treating prostate cancer, benign prostatic hyperplasia, acne, seborrhea, hirsutism, baldness, alopecia, precocious puberty, adrenal hypertrophy, polycystic ovary syndrome, breast cancer, endometriosis, lung cancer or leiomyoma.

20. Use of the compound according to claim 1 or a salt thereof for preventing or treating a disease associated with 17βHSD type 5.

21. Use of the compound according to claim 1 or a salt thereof for preventing or treating prostate cancer, benign prostatic hyperplasia, acne, seborrhea, hirsutism, baldness, alopecia, precocious puberty, adrenal hypertrophy, polycystic ovary syndrome, breast cancer, endometriosis, lung cancer or leiomyoma.

22. A method for treating and/or preventing a disease associated with 17βHSD type 5, comprising administering an effective amount of the compound according to claim 1 or a salt thereof to a patient.

23. A method for treating and/or preventing prostate cancer, benign prostatic hyperplasia, acne, seborrhea, hirsutism, baldness, alopecia, precocious puberty, adrenal hypertrophy, polycystic ovary syndrome, breast cancer, endometriosis, lung cancer or leiomyoma, comprising administering an effective amount of the compound according to claim 1 or a salt thereof to a patient.

24. An inhibitor of 17βHSD type 5, comprising the compound according to claim 1 or a salt thereof.

25. A commercial package, comprising a pharmaceutical composition containing the compound according to claim 1 or a salt thereof; and a description that the compound according to claim 1 or a salt thereof is capable of being used or should be used for treating and/or preventing prostate cancer, benign prostatic hyperplasia, acne, seborrhea, hirsutism, baldness, alopecia, precocious puberty, adrenal hypertrophy, polycystic ovary syndrome, breast cancer, endometriosis, lung cancer or leiomyoma.
